# EUROPEAN PATENT SPECIFICATION

(11) **EP 0 598 011 B1**
(45) Date of publication and mention of the grant of the patent: **11.11.1998**
(21) Application number: 92917507.3
(22) Date of filing: 03.08.1992
(51) Int. Cl.: C12N 15/85, C12N 15/00, C12N 15/62, C12N 15/12, C12N 15/38, C12N 15/63, C12N 15/31

(54) **ECDYSTEROID DEPENDENT REGULATION OF GENES IN MAMMALIAN CELLS**
ECDYSTEROID-ABHÄNGIGE GEN REGULIERUNG IN SÄUGETIER-ZELLEN
REGULATION DE GENES DANS DES CELLULES DE MAMMIFERE INDUITE PAR DES ECDYSTEROIDES

(30) Priority: 08.08.1991 US 742127
(43) Date of publication of application: 25.05.1994
(73) Proprietor: GENENTECH, INC., South San Francisco California 94080 (US)
(72) Inventor: GODOWSKI, Paul, J., Burlingame, CALIFORNIA 94010 (US)
(74) Representative: Barz, Peter, Dr.
(86) International application number: US9206391
(87) International publication number: WO9303162

(56) References cited:
- EP-A- 0 244 221
- WO-A-90/14356
- WO-A-91/13167
- PROCEEDINGS OF THE NATIONAL ACADEMY OF SCIENCES OF USA. vol. 89, July 1992, WASHINGTON US pages 6314 - 6318 CHRISTOPHERSON, K. S. ET AL. 'Ecdysteroid-dependent regulation of genes in mammalian cells by a Drosophila ecdysone receptor and chimeric transactivators'
- CELL, vol. 67, 4 October 1991, CAMBRIDGE, NA US, pages 59-77; KOELLE, M.R. ET AL.: 'The Drosophila EcR gene encodes an ecdysone receptor, a new member of the steroid receptor superfamily'

## Description

### FIELD OF THE INVENTION

This invention relates generally to the use of specific ecdysteroids in mammalian cells to induce gene expression of heterologous genes under the control of ecdysteroid receptor binding proteins.

### BACKGROUND OF THE INVENTION

The steroid hormone receptor superfamily represents an evolutionarily conserved group of proteins that influence developmental and metabolic processes primarily by functioning as ligand-dependent transcription factors (for review, see K. R. Yamamoto, *Annu. Rev. Genet.* **19***,* 209 [1985]; M. Beato, *Cell* **56**, 335 [1989]; R. M. Evans, *Science* **240**, 889 [1988]). Structural analysis of receptor proteins has identified domains of these proteins that function to bind DNA or ligand and to enhance transcription (S. Rusconi and K. R. Yamamoto, *EMBO J.* 6, 1309 [1987]; P. J. Godowski, D. Picard and K. R. Yamamoto, *Science* 241, 812 [1988]; S. M. Hollenberg and R. M. Evans, *Cell 55,* 899 [1988]; N. J. G. Webster, S. Green, J. R. Jin and P. Chambon, *Cell* **54**, 199 [1988]). Ligand binding is required for the interaction of some receptors with their cognate response elements (P. B. Becker, *et al., Nature* **324**, 686 [1988]) and may also control dimerization and transcriptional activation properties (S. Y. Tsai, *et al., Cell* **55**, 361 [1988); V. Kumar and P. Chambon, *Cell* 55, 145 [1988]).

The ability of transcription factors to function in heterologous species has provided a system to analyze the functional domains of that factor and a novel mechanism to control the expression of heterologous genes (A. J. Courey and R. Tjian, *Cell* **55**, 887 [1988); A. J. Courey, D. A. Holtzman, S. P. Jackson and R. Tjian, *Cell* **59**, 827 [1989]). Mammalian steroid hormone receptors have been shown to function when expressed in yeast (D. Metzger, J. H. White and P. Chambon, *Nature* **334**, 31 [1988); D. Picard, M. Schena and K. R. Yamamoto, *Gene* **86**, 257; M. Schena and K. R. Yamamoto, *Science* **241**, 965 [1988]) and drosophila cells.

The ecdysteroids are steroids whose action is mediated by an intracellular receptor and includes the molting hormones of insects. The ecdysteroid hormone 20-OH ecdysone, also known as β-ecdysone, controls timing of development in many insects. **See, generally,** Koolman (ed.), *Ecdysone: From Chemistry to Mode of Action,* Thieme Medical Pub., N.Y. (1989). The generic term "ecdysone" is frequently used as an abbreviation for 20-OH ecdysone. Pulses, or rises and falls, of the ecdysone concentration over a short period of time in insect development are observed at various stages of Drosophila development.

These stages include embryogenesis, three larval stages and two pupal stages. The last pupal stage ends with the formation of the adult fly. One studied effect of ecdysone on development is that resulting from a pulse at the end of the third, or last, larval stage. This pulse triggers the beginning of the metamorphosis of the larva to the adult fly. Certain tissues, called imaginal tissues, are induced to begin their formation of adult structures such as eyes, wings and legs.

During the larval stages of development, giant polytene chromosomes develop in the non-imaginal larval tissues. These cable-like chromosomes consist of aggregates comprising up to about 2,000 chromosomal copies. These chromosome aggregates are extremely useful because they provide the means whereby the position of a given gene within a chromosome can be determined to a very high degree of resolution, several orders of magnitude higher than is typically possible for normal chromosomes.

A "puff" in the polytene chromosomes is a localized expansion or swelling of these cable-like polytene chromosome aggregates that is associated with the transcription of a gene at the puff locus. A puff is, therefore, an indicator of the transcription of a gene located at a particular position in the chromosome.

A genetic regulatory model was proposed to explain the temporal sequence of polytene puffs induced by the ecdysone pulse which triggers the larval-to-adult metamorphosis. **See,** Ashburner *et al.,* "On the Temporal Control of Puffing Activity in Polytene Chromosomes," *Cold Spring Harbor Symp. Quant. Biol.* **38**:655-662 (1974). This model proposed that ecdysone interacts reversibly with a receptor protein, the ecdysone receptor, to form an ecdysone-receptor complex. This complex would directly induce the transcription of a small set of "early" genes responsible for a half dozen immediately induced 'early' puffs. These early genes are postulated to encode regulatory proteins that induce the transcription of a second set of "late" genes responsible for the formation of the "late" puffs that appear after the early puffs. The model thus defines a genetic regulatory hierarchy of three ranks, where the ecdysone receptor gene is in the first rank, the early genes in the second rank and the late genes in the third. While this model derived form the puffing pattem observed in a non-imaginal tissue, similar genetic regulatory hierarchies may also determine the metamorphic changes in development of the imaginal tissues that are also targets of ecdysone, as well as the changes in tissue development induced by the pulses of ecdysone that occur at other developmental stages.

Various structural data have been derived from vertebrate steroid and other lipophilic receptor proteins. A "superfamily' of such receptors has been defined on the basis of their structural similarities. **See,** Evans, "The Steroid and Thyroid Hormone Receptor Superfamily," *Science* **240**:889-895 (1988); Green and Chambon, "Nuclear Receptors Enhance Our Understanding of Transcription Regulation," *Trends in Genetics* **4**:309-314 (1988). Where their functions have been defined, these receptors, complexed with their respective hormones, regulate the transcription of their primary target genes, as proposed for the ecdysone receptor in the above model.

Ecdysteroid receptors from Drosophila melanogaster adult females were described by Handler *et al. Mol Cell Endo* **63**:103-9, 1989. Ecdysteroid receptors for the blowfly Calliphora vicina were characterized by Lehmann *et al, Eur J. Biochem***181**:577-82, 1989. The isolation of 20-hydroxyecdysone from Vitex strickeri (East African herb) is described in JP1135794. The ecdysterone content of culture medium of drosophila salivary glands was described in SU1130605. Muristerone extracted from the seeds of faladana plants and used as a biological insecticide is described in U.S. Patent 3,828,082. The synthesis of ecdysone is described in U.S. Patent 3,354,154.

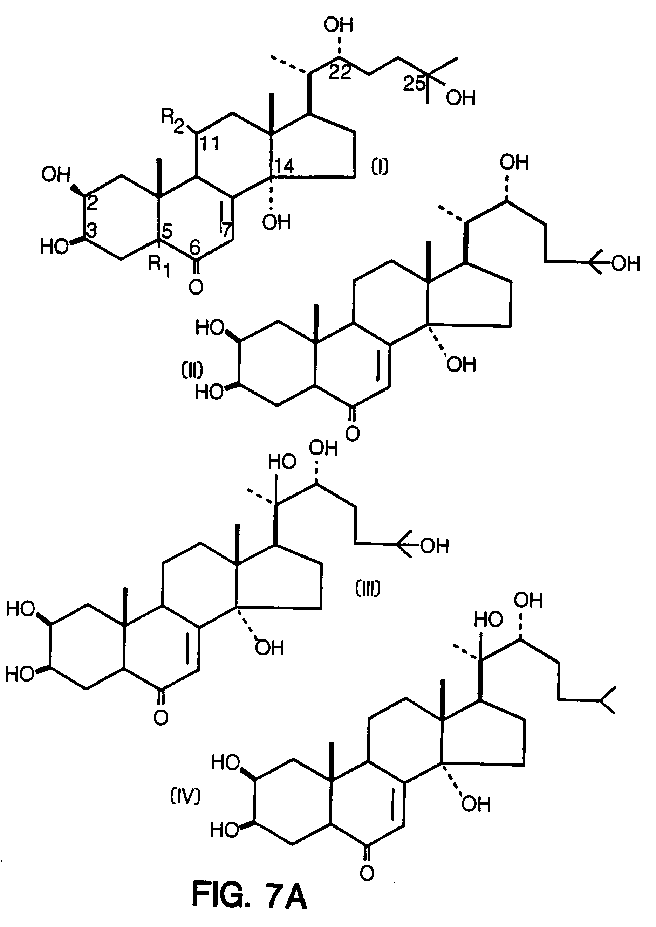

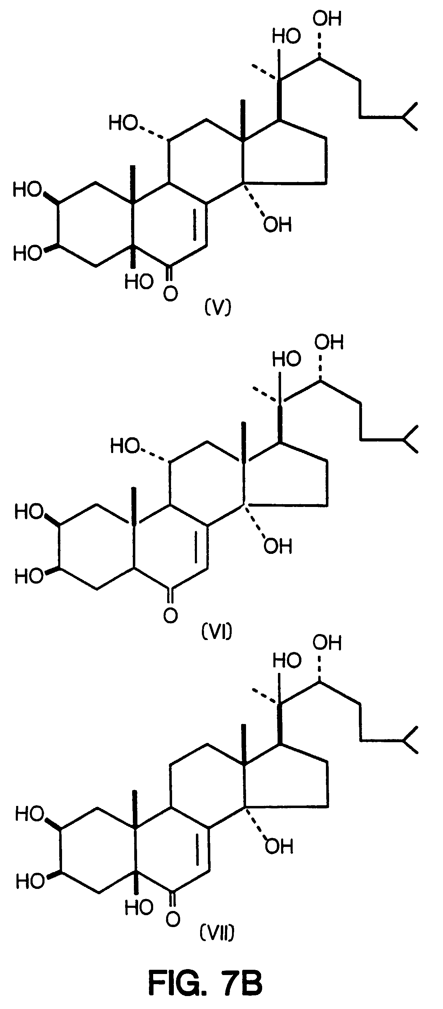

Analogs of ecdysteroids may be produced by plants to disrupt the development of insects. One plant-produced ecdysteroid analog is meristone A. Muristerone or Muristerone A (Figure 7, Compound IV) has a molecular weight of 496.640 daltons, a formula of C₂₇H₄₄O₈ and a CHCD name of 2,3,5,11,14,20,22-Heptahydroxy-7-cholesten-6-one (Cononica, L *et al, Phytochemistry* **14**: 525, 1975). It is a constitutient of the plant Ipomoea calonyction and shows a high insect moulting activity. It is believed to protect the plant by disrupting insect development in the larval stage (Trematerra *et al, Bollettino Zool. Agr. Bachic* **18**:87-93, [1986]). Muristerone A has been shown to have ecdysteroid activity in insects such as to trigger degeneration of the tick salivary gland (Lindsay *et al, J. Insect Physiol* **34**:351-360, [19881). Muristerone A-receptor complexes are not as sensitive to dissociation in high salt buffers as other ecdysteroid-receptor complexes and this affinity has allowed the use of radiolabeled muristerone A to follow ecdysteroid receptor during chromatography (Landon *et al.,* J. *Biol Chem* **263**:4693-4697, [1988]).

Many medically and commercially important proteins can be produced in a usable form by genetically engineered bacteria. However, many expressed proteins are processed incorrectly in bacteria and are preferably produced by genetically engineered eucaryotic cells. Typically, yeast cells or mammalian tissue-culture cells are used. Because it has been observed that protein processing of foreign proteins in yeast cells is also frequently inappropriate, mammalian cultured cells have become the central focus for protein production. It is common that the production of large amounts of foreign proteins makes these cells unhealthy, which may affect adversely the yield of the desired protein. This problem may be circumvented, in part, by using an inducible expression system. In such a system, the cells are engineered so that they do not express the foreign protein, and therefore are not unhealthy, until an inducing agent is added to the growth medium. In this way, large quantities of healthy cells can be produced and then induced to produce large amounts of the foreign protein. Unfortunately, in the presently available systems, the inducing agents themselves, such as metal ions or high temperature, adversely affect the cells, thus again lowering the yield of the desired foreign protein the cells produce. A need therefore exists for the development of innocuous inducing factors for efficient production of recombinant proteins. Such innocuous factors could also prove invaluable for human therapy, where the individual suffers from lack of the ability to produce particular proteins. By using methods similar to those for producing proteins in cultured cells, such innocuous factors for inducing the synthesis of the required protein could be used for controlling both the timing and the abundance of the protein produced in the affected individual. Therefore, a need exists for an inducible expression system in both mammalian cell culture and in mammals *per se.*

The hormones that complex with mammalian or other vertebrate members of the steroid receptor superfamily are unlikely candidates for such innocuous factors, nor have they been found to satisfy the required properties of such factors, because mammalian cells contain these receptors, or highly homologous proteins, that would alter the expression of many target genes in the presence of the respective hormone, thereby adversely affecting the host cells. For these and other reasons, developing an altemative steroid receptor system has been a goal of researchers. Unfortunately, efforts have been unsuccessful despite significant investment of resources. The absence of information on the structure, function and molecular biology of non-mammalian steroid receptors has significantly hindered the ability to produce such products. Recently, the isolation and characterization of drosophila DHR23α DNA, which contained a partial sequence of the DHR23α polypeptide, a member of the steroid hormone receptor superfamily previously identified in the laboratory of Dr. David Hogness (W. Seegraves, thesis, Stanford University [1988]) allows the isolation of the DNA sequence encoding the DHR23α. This isolated DNA sequence allows the construction of expression systems incorporating the DNA encoding the DHR23α ecdysteroid receptor.

In summary, the insect steroid hormones and their receptors, such as the ecdysteroids, are a potential source of material for developing innocuous steroid receptor systems for use in mammalian cell culture and in mammals themselves. However, no ecdysteroid has been shown to function to induce activity of an ecdysteroid receptor in a mammalian cell. Therefore, to develop a system based upon an insect hormone-receptor system, there exists a need for an ecdysteroid hormone or hormone analog that induces the ecdysteroid receptor in mammalian cells to express those genes placed under control of the ecdysteroid receptor.

### SUMMARY OF THE INVENTION

I show that DHR23α, a Drosophila steroid receptor homologue, can function in cultured mammalian cells as an ecdysteroid-dependent transcription factor when induced by a specific group of ecdysteroids which includes muristerone A. Muristerone A and related ecdysteroids that lack a 25-hydroxyl group, will induce in mammalian cells the expression of DNA sequences under the transcriptional control of DHR23α. DHR23α inducible activity was not induced by any of the mammalian steroid hormones tested. The DNA-binding and transactivation activities of viral, mammalian or bacterial proteins were rendered ecdysteroid-dependent when fused to the DHR23α ligand-binding domain. This system is useful in selectively regulating the expression of endogenous or heterologous genes in mammalian cells.

### DESCRIPTION OF THE FIGURES

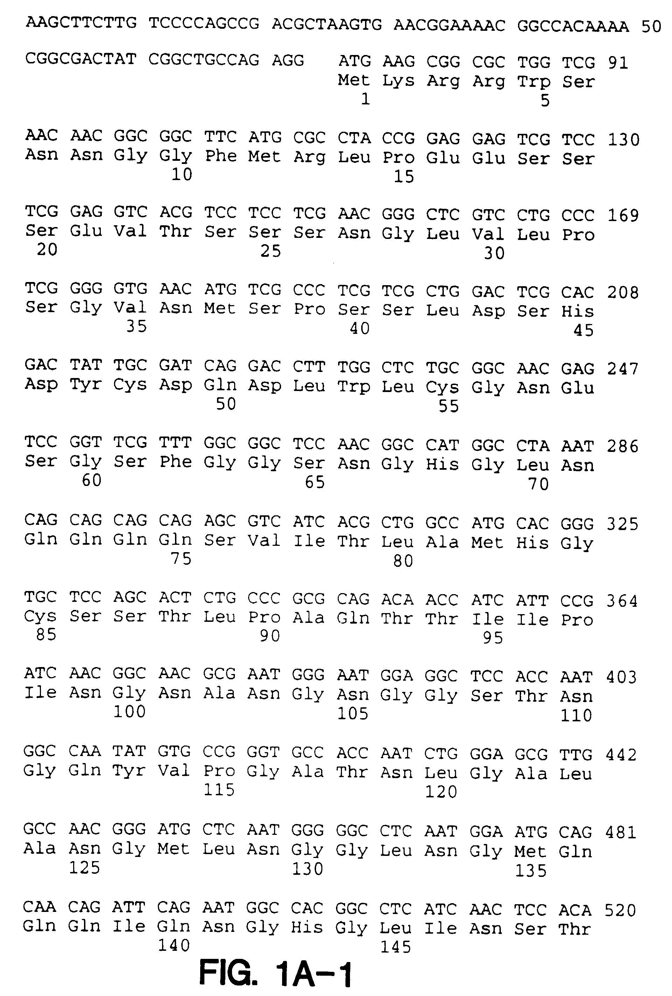

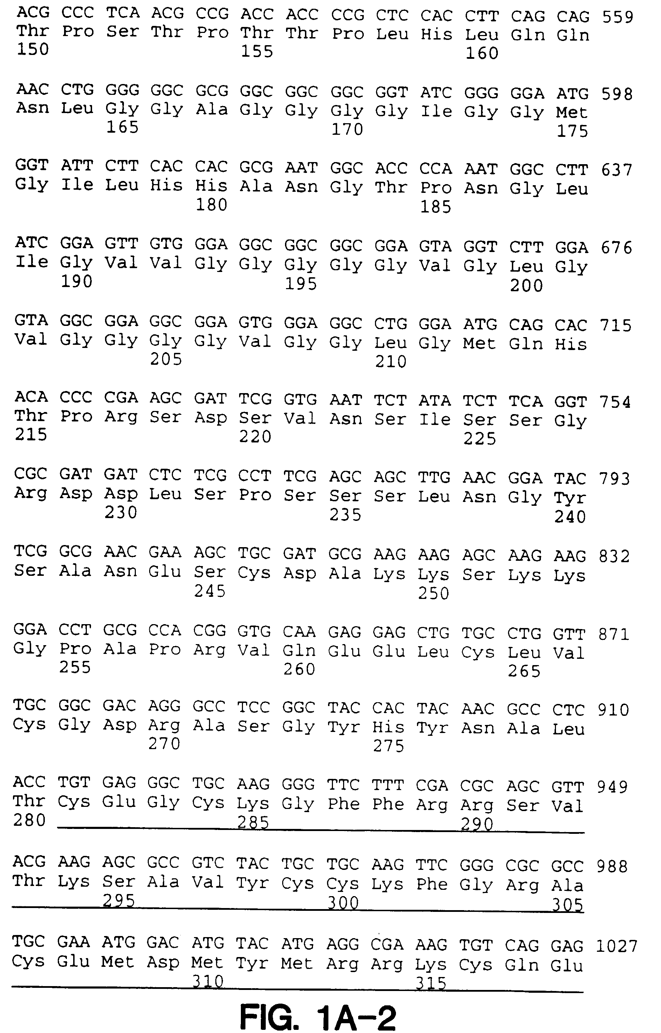

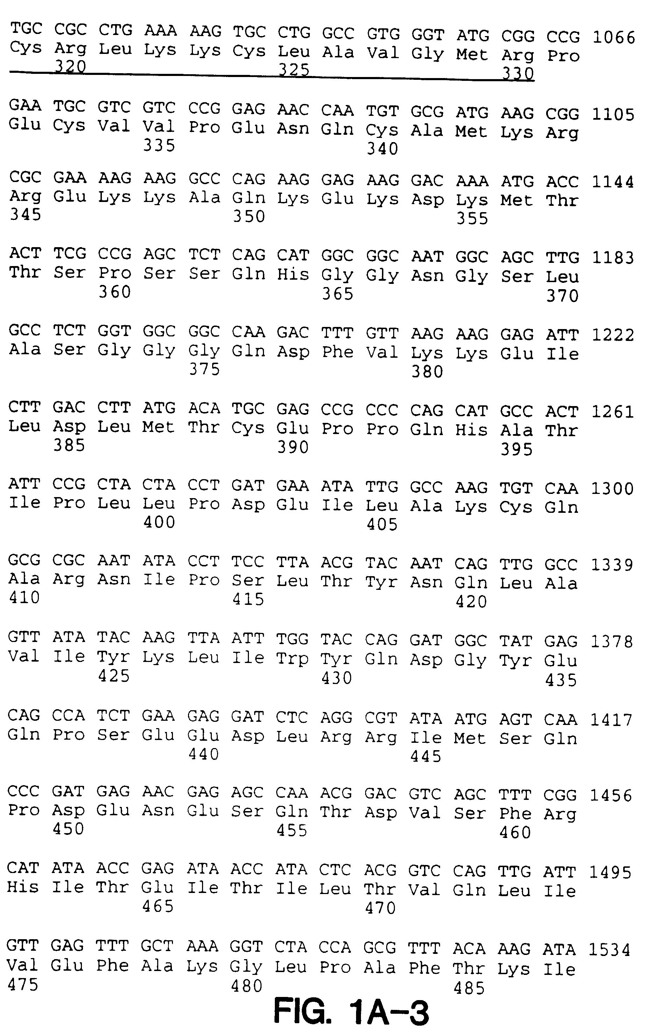

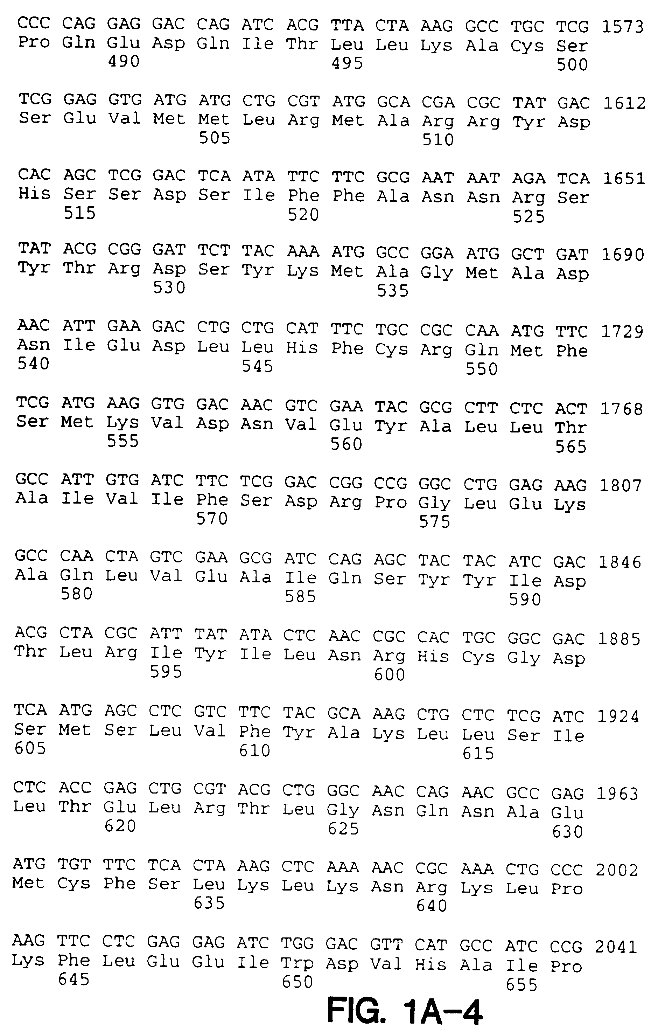

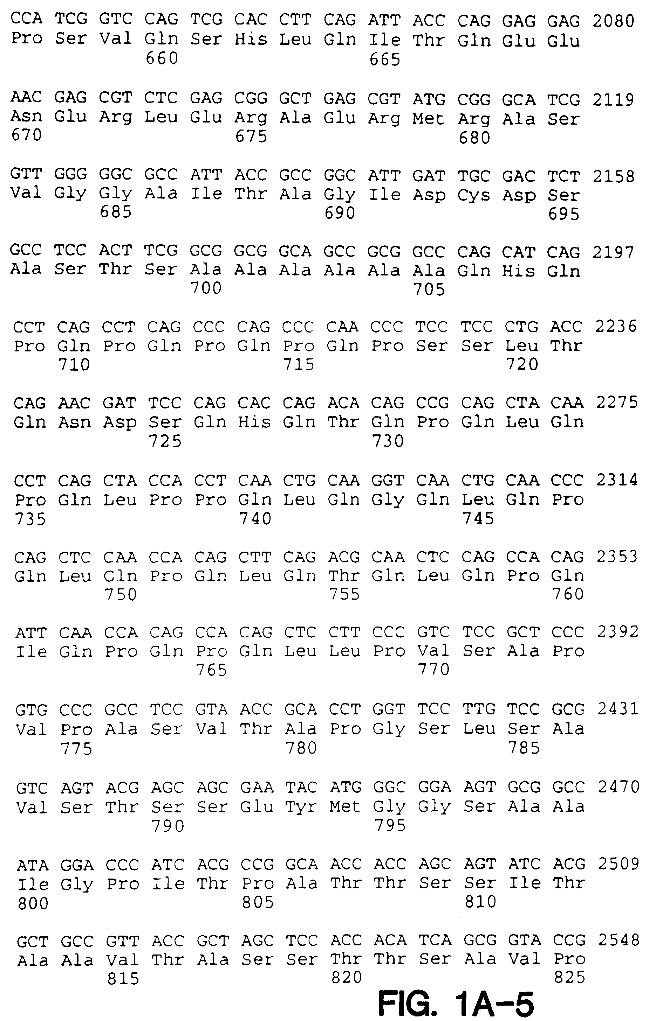

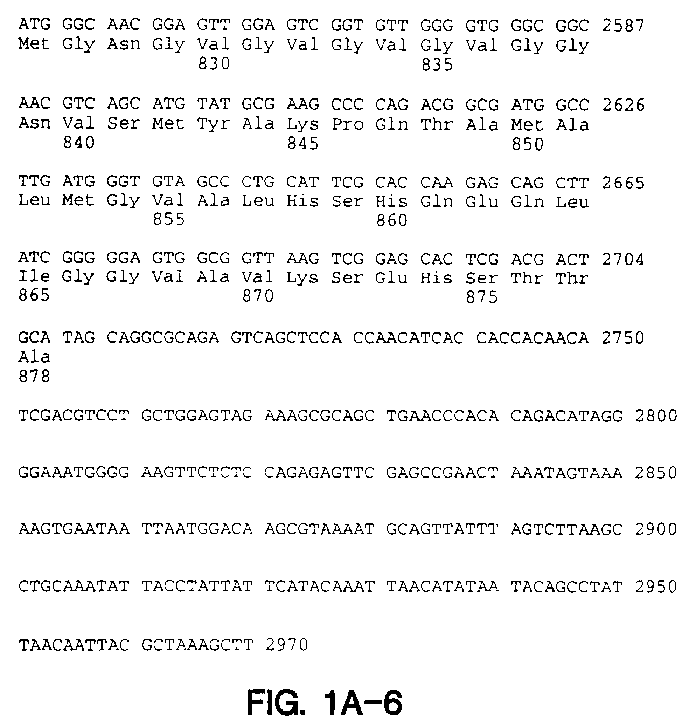

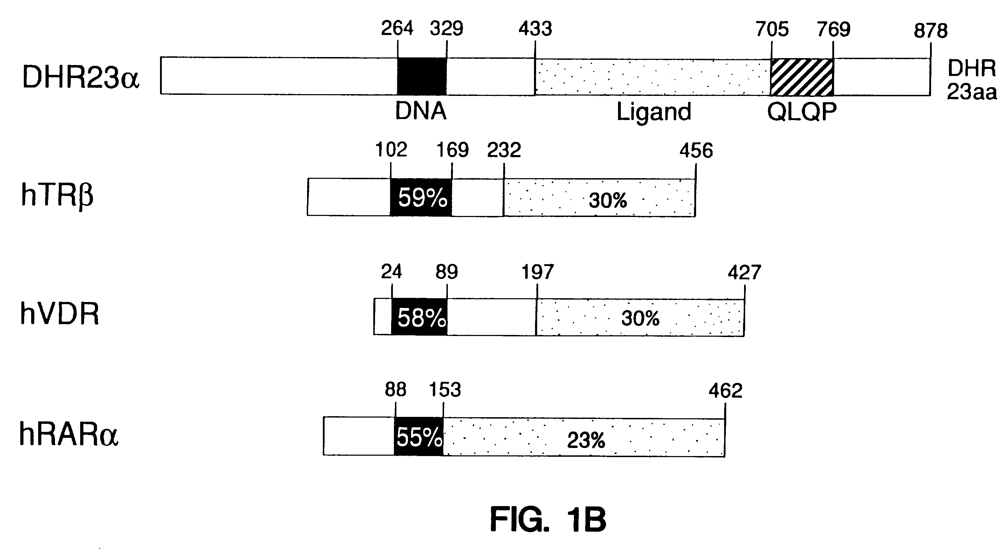

**Figure. 1.** (**A**) Nucleotide and derived amino acid sequence of the DHR23α cDNA clone (seq. ID No. 1). Numbers on the left and right indicate nucleotide and amino acid residues, respectively. The conserved amino acids corresponding to the putative DNA-binding domain are underlined. (B) Schematic comparison of the *Drosophila* DHR23α protein with the human thyroid hormone receptor (hTRβ), the human vitamin D3 receptor (hVDR) and the human retinoic acid receptor (hRARα). The amino acid residues are indicated by numbers above the boxes. The region of DHR23α marked 'DNA' is compared with the DNA-binding domains of the other receptors. The region of DHR23α marked 'Ligand' contains the highest region of homology to the ligand-binding domains of the other receptors. The percent identity in these regions is shown by the numbers within the boxes. The region labeled 'QLQP' is rich in the amino acids glutamine (Q) leucine (L) and proline (P).

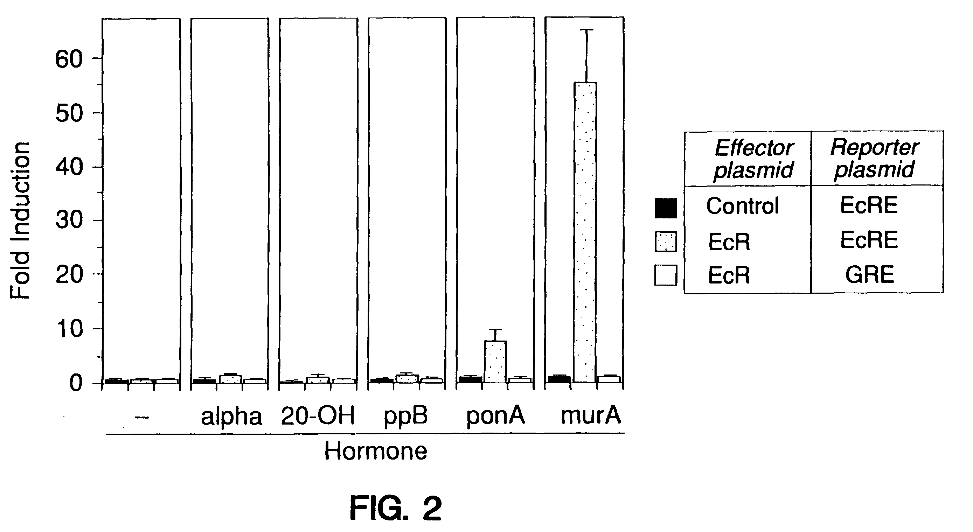

**Figure. 2.** Specific ecdysteroids are agonists for the DHR23α receptor in mammalian cells. Human 293 cells were cotransfected with 2.5 µg of the expression plasmid pRSV.DHR23α or the parental expression plasmid pRSV (Control) and 0.5 µgs of the reporter plasmid pEc₄M₋₇₇CO (EcRE) or pG₄M₋₇₇CO (GRE). As a control for transfection efficiency, 0.5 µgs of the control plasmid pRSV.hGH was included in the transfection mixture. After transfection, cells were treated without (-) or with alpha-ecdysone (alpha), 20-OH ecdysone (20-OH), polypodine B (ppB), ponasterone A (ponA) or muristeroine A (murA). CAT extracts were harvested 48 hrs after transfection and assayed. The values were normalized to the expression of hGH and the average values of three independent experiments are shown. The "fold induction" represents the level of expression of the reporter gene in cells incubated with hormone divided by the expression of that reporter gene in extracts from cells incubated in the absence of added ligand.

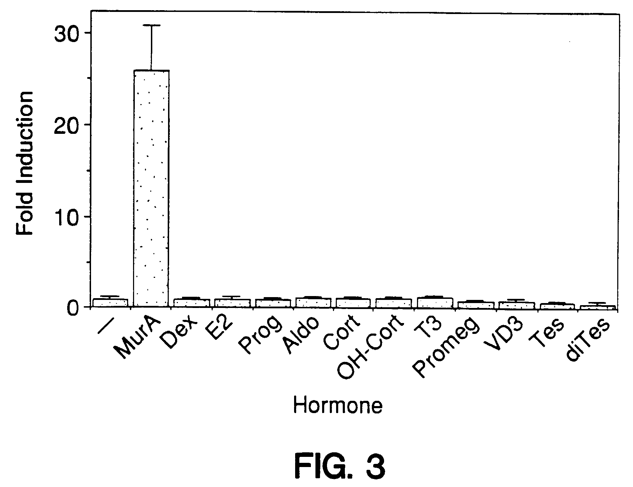

**Figure. 3.** Effect of mammalian hormones on activity of DHR23α. Cells were transfected with the DHR23α expression vector and Ec₄M₋₇₇CO reporter gene and treated without (-) or with the following hormones (1 µM): muristerone A (mur A) dexamethasone (Dex) 17β-estradiol (E2), aldosterone (Aldo), corticosterone (Cort) hydroxycorticosterone (OH-Cort) thyroid hormone (T3), promegestone (Promeg) or 1,25-dihydroxy vitamin D3 (VD3). Reporter gene activity was determined as described in Fig. 2.

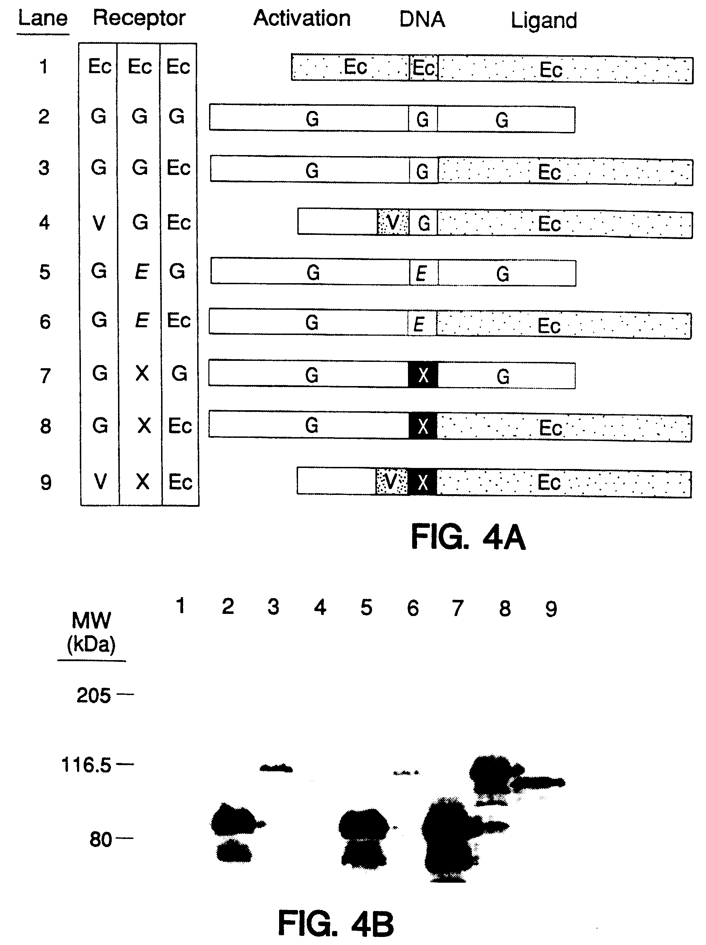

**Figure. 4.** Schematic representation and expression of receptor proteins. (**A**) Receptor constructs are denoted according to a three part nomenclature describing the origin of their N-terminal transactivation, DNA-binding and ligand-binding domains. "G" and "Ec" refer to the glucocorticoid receptor and DHR23α, respectively. '"E"' refers to a derivative of the rat glucocorticoid receptor DNA-binding domain with two amino acid substitutions (G458E, S459G) that convert the DNA-binding specificity to that of the estrogen receptor. "X" (solid box) indicates the DNA-binding domain (amino acidsl-87) of the *Escherichia coli* LexA protein. "V" (hatched box) denotes a derivative of the GR N-terminal domain in which amino acids 153-406 are replaced by the transcriptional activation domain of the HSV VP16 protein (amino acids 411-490) GGEc was constructed by replacing the ligand-binding domain of GGG (amino acids 528-795) with the ligand-binding domain of DHR23α (amino acids 329-878). Similarly, to construct GXEc, the ligand-binding domain of GXG was replaced with the DHR23α ligand-binding domain. (**B**) Accumulation of receptor proteins in transfected cells. Whole cell extracts were prepared 48 hrs after transfection with receptor expression plasmids encoding the following fusion proteins. Lane 1, EcEcEc; lane 2, GGG; lane 3, GGEc; lane 4, VGEc; lane 5, G"E"G; lane 6, G"E"Ec; lane 7, GXG; lane 8, GXEc; lane 9, VXEc. The blots were reacted with monoclonal antibody BµgR2 that recognizes an epitope in the N-terminal domain of the rat glucocorticoid receptor and then with a sheep antiserum to mouse antibody coupled to horseradish peroxidase. Positions of the molecular markers are indicated.

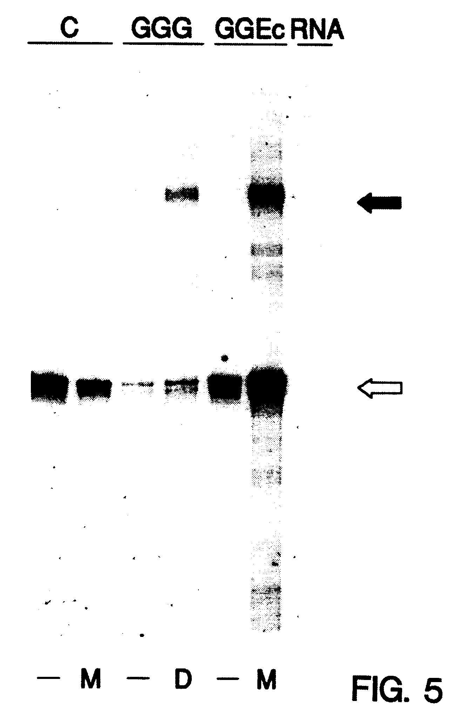

**Figure. 5.** RNase protection analysis of transcripts induced by receptor proteins. Total RNA was prepared from cells 48 hrs after transfection with expression plasmids encoding either DHR23a (EcEcEc), GGG, or GGEc and the reporter gene G₄M₋₇₇GO. The position of 377 base protected band for G₄M₋₇₇CO and the 294 base protected band from the intemal control gene (expressed from a CMV enhancer/promoter construct) are indicated by the closed and opened arrows, respectively.

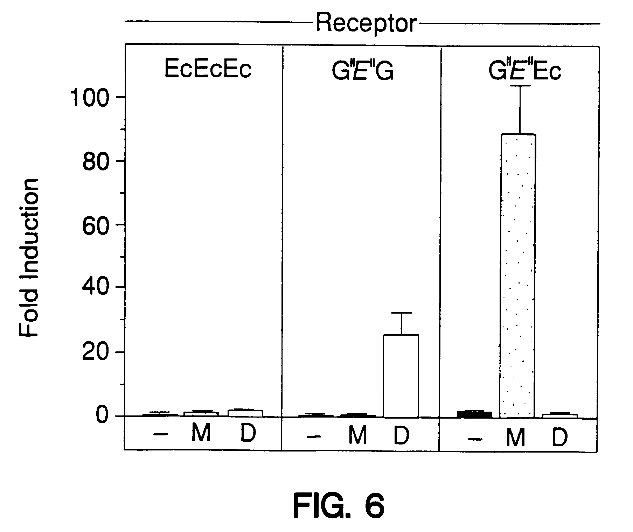

**Figure. 6.** Induction of estrogen receptors (EREs) by chimeric receptors. Cells were transfected with expression plasmids encoding eiher DHR23α (EcEcEc), G"E"G or G"E"Ec and E₄M₋₇₇CO, that contains 4 EREs fused to the MTV promoter. Cells were incubated for 48 hrs with or wihout (-) or with muristerone A (M) or dexamethasone (D).

**Figure 7.** The chemical structure of the ecdysteroids: (I) ecdysteroid numbering system, R₁ and R₂ are site of electronegative group substitution; (II) ecdysone; (III) 20-OH ecdysone(β-ecdysone); (IV) ponasterone A; (V) muristerone A; (VI) 5-dehydroxy muristerone A; (VII) and 11-dehydroxy muristerone A.

### DESCRIPTION OF THE PREFERRED EMBODIMENTS

Steroid receptors are members of a large family of transcription factors whose activity is tightly regulated by the binding of their cognate steroid ligand. These ligand-dependent transcription factors can be exploited to obtain the regulated expression of heterologous genes in mammalian cells. However, the utility of these systems in transgenic animals is limited by the background of endogenous steroids and their receptors. The present invention demonstrates that an analog of the insect ecdysteroids, muristerone A, will induce gene expression in mammalian cells using the insect steroid receptor DHR23α and its DNA binding sequence.

In the present invention, I wished to develop a system to specifically regulate heterologous genes in mammalian cells in tissue culture and in transgenic animals. Such a system provides a powerful method for regulating the synthesis of the products of heterologous genes. Systems that exploit the ability of mammalian steroid hormone receptors to function as ligand-dependent transcription factors have proved useful in regulating the expression of heterologous genes in mammalian cells (D. I. Israel and R. J. Kaufman, *Nuc. Acids Res.* **17**, 4589 [1989]). However, the applications of these systems in cultured cells or transgenic animals is limited by the background of endogenous steroid receptors and their ligands. In the present invention I show that the drosophila DHR23α protein, a member of the steroid hormone receptor superfamily previously identified in 1988 by W. Seegraves at Stanford University (W. Seegraves, thesis, Stanford University (1988]), can function as a ligand-dependent transcription factor in mammalian cells when induced by specific ecdysteroids such as DHR23α. The activity of DHR23α is induced upon administration of certain ecdysteroids but not by any of the mammalian hormones tested. Novel target gene specificity was obtained using chimeric receptors containing the DHR23α ligand-binding domains fused to heterologous DNA-binding domains. Finally, the activity of these chimeric proteins could be increased by inclusion of a potent viral transactivation domain.

Oligonucleotide probes based on the partial DHR23 sequence (W. Seegraves, thesis, Stanford University (1988]) were used to screen a cDNA library prepared from drosophila early pupal larvae. The deduced amino acid sequence of the DHR23α clone is shown in Fig. 1A. The highest region of homology between DHR23α and other members of the steroid receptor superfamily is found in a cysteine-rich region (residues 264-329) that corresponds to the DNA-binding domain (Fig. 1B). The putative ligand-binding domain shares limited homology with members of the retinoic acid and thyroid hormone receptors, and the vitamin D receptor. The N-terminal domain (residues 1-263) does not share significant homology with any steroid receptor superfamily member. I also identified a second form of this protein, DHR23β, that appears to arise by differential splicing. DHR23β is identical to DHR23α in the DNA and ligand-binding domains but contains an unrelated N-terminal domain of 234 amino acids.

DHR23α has been reported to regulate transcription of genes containing ecdysone response elements (EcREs) in *drosophila* tissue culture cells treated with 20-OH ecdysone (M. Koelle, Paper presented at a seminar, 2 October 1989, Genentech, Inc., So. San Francisco, CA). I determined whether DHR23α could function in mammalian cells treated with ecdysteroids to enhance the transcription of a reporter gene containing EcREs linked to the murine mammary tumor virus (MTV) promoter and chloramphenicol acetyltransferase (CAT) gene. Human 293 cells were cotransfected with an RSV-based expression vector that encodes DHR23α and the reporter gene Ec₄M₋₇₇CO that contains 4 copies of an ecdysone response element (EcRE) from the *Drosophila* HSP27 promoter (G. Riddihough and H. R. B. Pelham, *EMBO J.* 6, 3729 (1987]) linked to an MTV promoter-CAT construct. CAT activity was determined in extracts from cells incubated with or without the ecdysteroids α- or 20-OH ecdysone, polypodine B, ponasterone A, or muristerone A. Neither α-ecdysone, 20-OH ecdysone, nor polypodine B acted as agonists for DHR23α in mammalian cells. In contrast, expression of the reporter gene was markedly increased in cells treated with muristerone A and to a lesser extent with ponasterone A (Fig. 2). This induction was dependent on the presence of an EcRE in the reporter gene because DHR23a did not regulate expression of reporter genes containing binding sites for either the glucocorticoid receptor (GRE, Fig. 2) or for the estrogen receptor (ERE, Fig. 6). Thus, in mammalian cells DHR23α acts in an ecdysteroid-dependent fashion to selectively stimulate expression from an EcRE containing reporter gene.

It is unclear why 20-OH ecdysone and polypodine B, which are agonists in drosophila cell lines, fail to activate DHR23α in mammalian cells. Transport failure, inactivation and non-specific binding may account for the lack of activity in mammalian cells However, it has been shown that the specific activities and relative efficacies of ligands for their receptors sometimes differ when that receptor is expressed in a heterologous system. For example, it is known that the activities of glucocorticoid ligands for their receptor differ significantly in yeast and mammalian cells. Surprisingly, only specific derivatives of ecdysone act as agonists, and these share the related feature of lacking the 25-hydroxyl group. The only structural difference between the weak agonist ponasterone A and 20-OH ecdysone, which is inactive, is that the former lacks a hydroxyl group at position 25. Even more surprising and unexpected is that the strong agonist muristerone differs from ponasterone A solely by the addition of hydroxyl groups at positions 5 and 11. Therefore, surprisingly, in mammalian cells ecdysteroid inducers of DHR23α-regulated expression lack a hydroxyl group at position 25. In a preferred embodiment, the ecdysteroid may contain one hydroxyl group at positions 5 or 11. Altematively, any electronegative group substituted at positions 5 and/or 11 of muristerone would be expected to result in activation of ecdysteroid receptors. Among the electronegative groups that may be substituted at positions 5 and 11 of muristerone are hydroxyl, ketone, sulfhydral, nitrate, nitrite, and halogens, particularly florine, bromine and chlorine. The most active inducer of DHR23α regulated expression is muristerone A, which contains hydroxyl groups at both the 5 and 11 positions. The CHCD name for muristerone A is 2,3,5,11,14,20,22-heptahydroxy-7-cholesten-6-one; for 5-dehydroxy muristerone A it is 2,3,11,14,20,22-septahydroxy-7-cholesten-6-one; and for 11-dehydroxy muristerone A it is 2,3,5,14,20,22-septahydroxy-7-cholesten-6-one.

In the present invention, ecdysteroid receptor is defined as any insect steroid receptor that has a physiologically significant binding affinity for muristerone A, or a derivative of muristerone A which contains an electronegative substitution at the 5 or 11 positions. Among such physiologically significant receptors are the ecdysone receptor and DHR23X. A physiologically significant binding affinity is effective binding at a concentration of 10⁻⁶ molar or less. In the present invention, ecdysteroid is defined as any steroid lacking 25-OH having a physiologically effective affinity for an ecdysteroid receptor.

In order to determine if mammalian steroid hormones could act as agonists for DHR23a, I tested representative members of hormones known to activate mammalian steroid receptors. None of these hormones could act as agonists for DHR23α (Fig. 3). These results suggest that DHR23 activity is selectively regulated in mammalian cells by ecdysteroids. Therefore, mammalian cells containing genes under the control of DHR23α will be induced by muristerone A, and not by those mammalian steroid hormones tested.

### Ecdysteroid regulation of mammalian, viral and bacterial DNA binding or transactivation domains.

A remarkable feature of steroid hormone receptors is the degree to which individual domains can function when combined with domains of heterologous proteins. The DNA binding specificity of a receptor can be altered by replacing its DNA-binding domain with those of other steroid receptors (S. Green and P. Chambon, *Proc. Natl. Acad. Sci. U.S.A.* **325**, 75 [1987]) or from bacterial (P. J. Godowski, D. Picard and K. R. Yamamoto, *Science* **241**, 812 (1988)) or yeast (N. J. G. Green, S. Green, J. R. Kin and P. Chambon, *Cell* **54**, 199 [1988]) DNA-binding proteins. In some cases, the activities of heterologous proteins become hormone regulated if that protein is fused to to a steroid receptor ligand-binding domain. For example, the transactivation or transformation activities of *E1A, c-myc* or *c-fos* can be brought under hormonal control by fusion of a steroid receptor ligand-binding domain (M. Eilers, D. Picard, K. R. Yamamoto and J. M. Bishop, *Nature* **340**, 66 [1989]; D. Picard, S.J. Salser and K. R. Yamamoto, *Cell* **54**, 1073 [1988]; G. Superti-Furga, G. Bergers, D. Picard and M. Busslinger, *Proc. Natl. Acad. Sci. U.S.A.* **88**, 5114 [1991]).

I choose to determine if the ligand-binding domain of DHR23a could be used to regulate the DNA-binding and transactivation domains of the mammalian glucocorticoid receptor for the following reasons. First, in contrast to DHR23α, high affinity DNA-binding sites for the GR have been identified (M. Beato, *Cell* **56**, 335 [1989]) and reporter genes containing these sites are very strongly regulated. Thus, the sensitivity of our assays could be increased. Secondly, these chimeric receptors could be utilized to selectively regulate endogenous genes in mammalian cells in response to ecdysteroids. I constructed a chimeric gene, GGEc, in which the sequences encoding the ligand-binding domain of the GR were replaced with that of DHR23α (Fig. 4A). Western blot analysis indicated that GGEc was expressed in transfected cells, although at considerably lower levels than the intact GR (Fig. 4B). As expected, DHR23α failed to induce the expression of G₄M₋₇₇CO, a reporter gene that contains 4 copies of a high affinity GRE (Table 1). However, expression of the GRE-containing reporter gene was induced more than 700 fold by GGEc in muristerone-treated cells. The relative efficacies of ecdysteroids as agonists for the chimeric receptor are identical to that as for DHR23α; neither α-ecdysone, 20-OH ecdysone or polypodine B acted as agonists whereas ponasterone A and muristerone A acted as weak and strong agonists respectively.

Results of the CAT assays were confirmed and extended by direct analysis of transcripts initiated at the regulated promoter. RNA isolated from transfected cells was assayed by RNAse mapping experiments using probes complementary to the GRE containing reporter plasmid and a cotransfected control gene expressed from the CMV enhancer and promoter. Figure 5 shows that GGEc acts in a hormone-dependent fashion to stimulate expression from a GRE-MTV promoter construct.

I then determined if a DHR23 fusion protein could regulate genes normally responsive to estrogens. I constructed a derivative of GGEc that incorporates a two amino acid change in the first finger of the rat glucocorticoid receptor (G458E, S459G) that has been shown to convert the DNA-binding specificity of the GR to that of the ER (K. Umesono and R. Evans, *Cell* **57,** 1139 [1989]; M. Danielsen, L. Hinck, G. Ringold, *Cell* **57,** 1131 [1989]) (Fig. 4A). This fusion protein, G"E"Ec, now regulates the ERE containing reporter gene E4M-77CO in a muristerone-dependent fashion (Figure 6). As expected, G"E"Ec failed to induce the expression of reporter genes lacking EREs

Chimeric proteins containing the DHR23α ligand-binding domain fused to the DNA-binding domains of mammalian proteins are expected to prove useful in regulating the expression of either native or transgenic endogenous genes in transgenic animals. Such fusion constructs are expected to be useful in regulating the expression of exogenous genes introduced into transgenic mammals or mammalian cells. I next determined if the DHR23α ligand-binding domain could be used to regulate the activity of a DNA-binding domain not normally expressed in mammalian cells. I constructed the chimeric gene GLxEc by replacing the sequences coding for the GR DNA-binding domain in the GGEc fusion with those encoding the DNA-binding domain of the *Escherichia coli* LexA repressor (J. W. Little and S. A. Hill, *Proc. Natl. Acad. Sci. U.S.A.* **82**, 2301 [1985]) (Fig. 4). A reporter gene, X₄C₋₃₃CO was constructed that contains 4 copies of a 26-bp lex operator (R. Brent and M. Ptashne, *Nature* **312**, 612 [1984]) at position -33 of the CMV promoter. GLxEc had no effect on the expression of a OC₋₃₃CO, a reporter gene lacking the lex operator (Fig. 6B). However, transcription of X₄C₋₃₃CO was strongly induced by GLxEc, and this induction was fully hormone-dependent (Fig. 6A). As controls I showed that X₄C₋₃₃CO was not induced in cells treated with muristerone and cotransfected with either DHR23α, that lacks the lexA DNA-binding domain, or by GLxG (P. J. Godowski, D. Picard and K. R. Yamamoto, *Science* **241**, 812 [1988]) that contains the glucocorticoid receptor ligand-binding domain.

Finally, I determined if the activity of DHR23α fusion proteins could be further enhanced by inclusion of a potent viral transactivation domain. I constructed VGEc and VLxEc fusion genes by replacing a portion of the GR N-terminal activation domain in GGEc and GLxEc, respectively, with the herpes virus VP16 acidic activation domain (I. Sadowski *et al., Proc. Natl. Acad. Sci.* **335**, 563 [1988]; D. J. Cousens, R. Greaves, C. R. Coding, and P. O'Hare, *EMBO* J. **8**, 2337 [1989]) (Fig. 4A). In transfected cells, these proteins accumulated to similar levels as derivatives lacking the VP16 activation domain (Fig. 4B). Both VGEc and VLxEc acted in an ecdysteroid-dependent fashion to induce activity of the appropriate reporter gene (Tables 1 and 2). However, the activity of VGEc and VLxEc was 5 and 10 fold greater than GGEc and GLxEc, respectively (M..A. Labow, S.B. Baim, T. Shenk, and A. J. Levine, *Mol. and Cell. Biol.* **10**, 3343 [1990]). Thus, the DHR23α ligand-binding domain can be used to regulate the activities of viral, mammalian and bacterial DNA-binding or transactivation domains.

The development of a system for regulated expression of endogenous and exogenous genes in eukaryotic cells provides an important method to study the function of those gene products and to develop animals models for disease. Our results demonstrate the feasibility of using nonmammalian steroid hormone receptors to regulate genes in mammalian cells. There are several important features of this system. DHR23α acts as a potent and selective regulator of the transcription of genes containing EcREs', and the activity of DHR23α can be further modified by replacing its DNA-binding or transactivation domains with those from heterologous proteins. Importantly, DHR23α activity is regulated by ecdysteroids, which are not normally expressed in mammalian cells. Although I did not survey all of the mammalian steroids, none of those representing the most abundant of the natural murine steroids acted as agonists for DHR23α. Thus, it is conceivable that the transcriptional regulatory activities of DHR23α or DHR23α fusion proteins will be completely dependent on administration of exogenous ligand.

Several reports have demonstrated the feasibility of using the *E. coli* lac repressor to regulate gene expression in mammalian cells. Both the lac repressor and the steroid receptor based systems can induce or repress (D. M. Omitz, R. W. Moreadith and Leder P., *PNAS* **88**, 698 [1991]) transcription. One attractive feature of a steroid receptor based regulatory system is the remarkable flexibility in the types of activities that can be controlled by the ligand-binding domain. The activities of structurally distinct DNA-binding proteins such as *lexA, c-fos,* GAL4 and *c-myc* are rendered hormone-dependent when fused to a steroid receptor ligand-binding domain (P. J. Godowski, D. Picard and K. R. Yamamoto, *Science* **241**, 812 [1988]; N. J. G. Green, S. Green, J. R. Kin and P. Chambon, *Cell* **54**, 199 [1988]; M. Eilers, D. Picard, K. R. Yamamoto and J. M. Bishop, *Nature* **340**, 66 [1989]; D. Picard, S.J. Salser and K. R. Yamamoto, *Cell* **54**, 1073 [1988]; G. Superti-Furga, G. Bergers, D. Picard and M. Busslinger, *Proc. Natl. Acad. Sci. U.S.A.* **88**, 5114 [1991]). Chimeric proteins can be further modified by addition or subtraction of transactivation domains. Thus, it is expected that DHR23a fusion genes can be constructed to regulate the expression of virtually any gene for which a cis-acting regulatory sequence and its cognate DNA-binding domain have been identified.

Among the genes that are anticipated for use in the method of the present invention are those encoding cytokines, hormones, structural proteins, enzymes and nucleic acids that are are present in less than therapeutically needed concentrations in mammals. Among the cytokines and hormones are polypeptides such as: growth hormone, insulin-like growth factors, interleukins, human growth hormone, N-methionyl human growth hormone, bovine growth hormone, parathyroid hormone, thyroxine, insulin, proinsulin, relaxin, prorelaxin, glycoprotein hormones such as follicle stimulating hormone FSH), thyroid stimulating hormone (TSH), parathyroid hormone, and leutinizing hormone (LH), hemopoietic growth factor, hepatic growth factor, fibroblast growth factor, prolactin, placental lactogen, tumor necrosis factor-alpha and -beta, mullerian-inhibiting substance, mouse gonadotropin-associated peptide, glucagon, inhibin, activin, vascular endothelial growth factor, integrin, thrombopoietin, erythropoietin, nerve growth factors such as NGF-β, platelet-growth factor, hemopoietic growth factor, tissue factor protein, mullerian-inhibiting substance, mouse gonadotropin-associated peptide, transforming growth factors (TGF) such as TGF-alpha and TGF-beta, insulin-like growth factor-I and -II, latency associated peptide, erythropoietin, osteoinductive factors, osteoinductive factors, interferons such as interferon-alpha, -beta, and -gamma, colony stimulating factors (CSFs) such as M-CSF, GM-CSF, and G-CSF, interleukins (ILs) such as IL-1, IL-2, IL-3, IL-4, IL-5, IL-6, IL-7, IL-8 and other polypeptide factors.

Among the enzymes are tissue plasminogen activase (TPA), urokinase, elastase, adenosine deaminase, bombesin, factor IX, factor VIII, thrombin, enkephalinase, β-lactamase, superoxide dismutase, reverse transcriptase, RNase, DNase, enzymes involved in glycolysis, Kreb's cycle, gluconeogenesis, urea cycle, oxidative phosphorylation, the synthesis or degradation of purines, pyrimidines, nucleic acid polymers, cholesterol, protein, glycogen, fatty acids, carbohydrate, phospholipids, glycoproteins or lipids.

Among the structural proteins are lung surfactant, albumin, antibodies, carrier proteins, glycoprotein hormone receptors, CD-4, insulin-like growth factor binding proteins, calcitonin, factor VIII, an antibody, protein A or D, rheumatoid factors, viral antigens, HIV envelope proteins GP120 and GP140, immunoglobulins, serum proteins and any protein present in a suboptimal amount.

Among the nucleic acids are DNA and RNA, both sense and antisense strands. The RNA may be any type, including ribosomal RNA, messenger RNA, transfer RNA, small nuclear RNA and RNA which has an enzymatic function or participates in an enzymatic function. The methods of the present invention have application to the production of nucleic acids which act as antisense or suppressor nucleic acids and inhibit the activity of a naturally occurring mammalian nucleic acid. Such nucleic acids have particular therapeutic efficacy in treating viral diseases, cancers and excessive production of a protein or other metabolic product.

Omitz, *et al.* (D. M. Omitz, R. W. Moreadith and Leder P., *PNAS*_**88**, 698 ([1991]) have described a binary system for regulating expression of heterologous genes in transgenic mice. In this system, a "transactivator" strain expressing the yeast GAL4 protein is crossed with "target" strains containing a transcriptionally silent transgene controlled by UAS sequences. The bigenic progeny of this cross express both transgenes in the same tissue. Bigenic systems incorporating DHR23α or DHR23α gene-fusions will provide a general method to control the abundance, time course and/or tissue specific expression of endogenous or exogenous genes. For example, tissue specific and developmentally regulated expression of transgenes controlled by EcREs is expected to be achieved by targeting the expression DHR23α with appropriate tissue specific enhancers. The expression of the EcRE containing "target" gene is expected to be induced at appropriate times by "turning-on" its activator with ecdysteroids. This development of a system that allows tissue and developmental control of genes in transgenic animals provides an important approach that complements strategies based on "gene-knockout" technology.

Mammalian cells suitable for the present method include those contained in an intact mammal. They include both differentiated and undifferentiated cells. Also included within the term mammalian cells are established cell lines and primary cell cultures derived from mammalian tissue. Among the preferred mammalian cell lines are 293 cell line and CHO cell line.

### Therapeutic Compositions and Administration of Ecdysteroid

Ecdysteroids may be administered to transgenic mammals or mammalian cells that contain a gene under the transcriptional control of an ecdysteroid. For example, gene therapy to enable a mammal to express a polypeptide previously produced in insufficient quantities. Examples of such polypeptides are described above.

Therapeutic formulations of ecdysteroids are prepared for storage by mixing ecdysteroid subunit having the desired degree of purity with optional physiologically acceptable carriers, excipients, or stabilizers *(Remington's Pharmaceutical Sciences, supra),* in the form of lyophilized cake or aqueous solutions. Acceptable carriers, excipients or stabilizers are nontoxic to recipients at the dosages and concentrations employed, and include buffers such as phosphate, citrate, and other organic acids; antioxidants including ascorbic acid; low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as Tween, Pluronics or polyethylene glycol (PEG).

The ecdysteroid to be used for *in vivo* administration must be sterile. This is readily accomplished by filtration through sterile filtration membranes, prior to or following lyophilization and reconstitution. The ecdysteroid ordinarily will be stored in lyophilized form or in solution. Therapeutic ecdysteroid compositions generally are placed into a container having a sterile access port, for example, an intravenous solution bag or vial having a stopper pierceable by a hypodermic injection needle.

The route of ecdysteroid administration is in accord with known methods, e.g. injection or infusion by intravenous, intraperitoneal, intracerebral, intramuscular, intraocular, intraarterial, or intralesional routes, or by sustained release systems as noted below. The ecdysteroid is administered continuously by infusion or by bolus injection.

Suitable examples of sustained-release preparations include semipermeable matrices of solid hydrophobic polymers containing the ecdysteroid, which matrices are in the form of shaped articles, e.g. films, or microcapsules. Examples of sustained-release matrices include polyesters, hydrogels (e.g., poly(2-hydroxyethyl-methacrylate) as described by Langer *et al.,* J*. Biomed. Mater. Res.* **15**: 167-277 (1981) and Langer, *Chem. Tech.* **12**: 98-105 (1982) or poly(vinylalcohol), polylactides (U.S. 3,773,919, EP 58,481), copolymers of L-glutamic acid and gamma ethyl-L-glutamate (Sidman *et al., Biopolymers* **22**: 547-556 [1983]), non-degradable ethylene-vinyl acetate (Langer *et al., supra),* degradable lactic acid-glycolic acid copolymers such as the Lupron Depot™ (injectable micropheres composed of lactic acid-glycolic acid copolymer and leuprolide acetate), and poly-D-(-)-3-hydroxybutyric acid (EP 133,988).

Sustained-release ecdysteroid compositions also include liposomally entrapped ecdysteroid. Liposomes containing ecdysteroid are prepared by methods known *per se:* DE 3,218,121; Epstein *et al., Proc. Natl. Acad. Sci.* **82**: 3688-3692 (1985); Hwang *et al., Proc. Natl. Acad. Sci.* **77**: 4030-4034 (1980); EP 52,322; EP 36,676; EP 88,046; EP 143,949; EP 142,641; Japanese patent application 83-118008; U.S. 4,485,045 and 4,544,545; and EP 102,324. Ordinarily the liposomes are of the small (about 200-800 Angstroms) unilamelar type in which the lipid content is greater than about 30 mol. % cholesterol, the selected proportion being adjusted for the optimal ecdysteroid therapy.

An effective amount of ecdysteroid to be employed therapeutically will depend, for example, upon the therapeutic objectives, the route of administration, the activity of the polypeptide induced by the ecdysteroid and the condition of the patient. Accordingly, it will be necessary for the therapist to titer the dosage and modify the route of administration as required to obtain the optimal therapeutic effect. A typical daily dosage might range from about 1 µg/kg to up to 100 mg/kg or more, depending on the factors mentioned above. Typically, the clinician will administer the ecdysteroid until a dosage is reached that achieves the desired effect. The progress of this therapy is easily monitored by conventional assays for the presence or activity of the induced polypeptide.

Ecdysteroid compositions are administered to transgenic mammals, or to transgenic mammalian cell culture, when there is within the target cell population an available ecdysteroid receptor and an ecdysteroid DNA-binding domain positioned to promote the expression of a DNA sequence encoding a desired polypeptide or nucleic acid. The ecdysteroid receptor, such as DHR23α, acts as a potent and selective regulator of the transcription of genes containing ecdysteroid receptor DNA-binding sites. The activity of ecdysteroids, such as DHR23α, can be further modified by replacing its DNA-binding or transactivation domains with those from other naturally occurring genes, thereby facilitating ecdysteroid control over the other naturally occurring genes and the products they produce. The chimeric ecdysteroid receptors still bind the same ligand, however, they induce expression of the gene specified by the heterologous DNA binding domain specified by fused transactivation domain. Transformation of mammalian cells is accomplished using standard methods. Vectors suitable for transforming mammalian cells include viral and mammalian DNA capable of expression in mammalian cells.

The following examples are offered by way of illustration and not by way of limitation.

### EXAMPLE 1

### CLONING OF DNA ENCODING DHR23α

Oligonucleotide probes based on the partial DHR23 sequence (W. Seegraves, thesis, Stanford University [1988]) were used to screen a cDNA library prepared from drosophila early pupal larvae. The deduced amino acid sequence of the DHR23α clone is shown in Fig. 1A. The highest region of homology between DHR23α and other members of the steroid receptor superfamily is found in a cysteine-rich region (residues 264-329) that corresponds to the DNA-binding domain (Fig. 1B). The putative ligand-binding domain shares limited homology with members of the retinoic acid and thyroid hormone receptors, and the vitamin D receptor. The N-terminal domain (residues 1-263) does not share significant homology with any steroid receptor superfamily member. I also identified a second form of this protein, DHR23β, that appears to arise by differential splicing. DHR23β is identical to DHR23α in the DNA and ligand-binding domains but contains an unrelated N-terminal domain of 234 amino acids.

Figure 1 A illustrates the nucleotide and derived amino acid sequence of the DHR23α cDNA clone. Numbers on the left and right indicate nucleotide and amino acid residues, respectively. The first ATG following an upstream, in-frame stop codon is underlined and was chosen as the initiating codon. The initiator methionine conforms to the *Drosophila* consensus sequence for translation initiation. The conserved amino acids corresponding to the putative DNA-binding domain are underlined. To isolate the full-length DHR23α clones, -600,000 phage from a *Drosophila* third instar larval library were screened with two 50-mer oligonucleotide probes corresponding to nucleotides 109-158 and 1820-1869 of the partial DHR23α sequence in Seegraves, Stanford University Thesis, 1988. Eight positive clones were isolated, further characterized by PCR analysis and the nucleotide sequence of the two largest inserts was determined. The results of the sequencing are shown in figure 1A.

### EXAMPLE 2

### EXPRESSION AND ACTIVITY OF DHR23α POLYPEPTIDE IN MAMMALIAN CELLS

I determined whether DHR23α could function in mammalian cells treated with ecdysteroids to enhance the transcription of a reporter gene containing EcREs linked to the murine mammary tumor virus (MTV) promoter and chloramphenicol acetyltransferase (CAT) gene. Human 293 cells were cotransfected with an RSV-based expression vector that encodes DHR23a and the reporter gene Ec₄M₋₇₇CO that contains 4 copies of an ecdysone response element (EcRE) from the *Drosophila* HSP27 promoter (G. Riddihough and H. R. B. Pelham, *EMBO J.* **6**, 3729 [1987]) linked to an MTV promoter-CAT construct. CAT activity was determined in extracts from cells incubated with or without the ecdysteroids α- or 20-OH ecdysone, polypodine B, ponasterone A, or muristerone A. Neither α-ecdysone, 20-OH ecdysone, nor polypodine B acted as agonists for DHR23α in mammalian cells. In contrast, expression of the reporter gene was markedly increased in cells treated with muristerone A and to a lesser extent with ponasterone A (Fig. 2). This induction was dependent on the presence of an EcRE in the reporter gene because DHR23α did not regulate expression of reporter genes containing binding sites for either the glucocorticoid receptor (GRE, Fig. 2) or for the estrogen receptor (ERE, Fig. 6). Thus, in mammalian cells DHR23α acts in an ecdysteroid-dependent fashion to selectively stimulate expression from an EcRE containing reporter gene.

Specific ecdysteroids are agonists for the DHR23α receptor in mammalian cells. Human 293 cells were cotransfected with 2.5 µg of the expression plasmid pRSV.DHR23α or the parental expression plasmid pRSV (Control) and 0.5 µgs of the reporter plasmid pEc₄M₋₇₇CO (EcRE) or pG₄M₋₇₇CO (GRE). As a control for transfection efficiency, 0.5 µgs of the control plasmid pRSV.hGH was included in the transfection mixture. After transfection, cells were treated without (-) or with alpha-ecdysone (alpha), 20-OH ecdysone (20-OH), polypodine B (ppB), ponasterone A (ponA) or muristeroine A (murA). All candidate ligands were added at a concentration of 1 µM. CAT extracts were harvested 48 hrs after transfection, and assays were performed as described (D. R. Cavener, *Nucl. Acids Res.* **15**, 1353-1361 [1987]). The values were normalized to the expression of hGH and the average values of three independent experiments are shown in Figure 2. The "fold induction" represents the level of expression of the reporter gene in cells incubated with hormone divided by the expression of that reporter gene in extracts from cells incubated in the absence of added ligand.

The effect of mammalian hormones on activity of DHR23α is shown in Figure 3. Cells were transfected with the DHR23α expression vector and Ec₄M₋₇₇CO reporter gene and treated without (-) or with the following hormones (1 µM): muristerone A (mur A) dexamethasone (Dex) 17β-estradiol (E2), aldosterone (Aldo), corticosterone (Cort) hydroxycorticosterone (OH-Cort) thyroid hormone (T3), promegestone (Promeg) or 1,25-dihydroxy vitamin D3 (VD3). Reporter gene activity was determined as described above for Figure 2. Retinoic Acid also did not act as an agonist for DHR23α.

A schematic representation of chimeric genetic constructs and a Westem blot of the resulting expressed receptor proteins are illustrated in figures 4A and 1B. In Figure 4A receptor constructs are denoted according to a three-part nomenclature describing the origin of their N-terminal transactivation, DNA-binding and ligand-binding domains. "G" and "Ec" refer to the glucocorticoid receptor and DHR23α, respectively. "E" refers to a derivative of the rat glucocorticoid receptor DNA-binding domain with two amino acid substitutions (G458E, S459G) that convert the DNA-binding specificity to that of the estrogen receptor (K. Umesono and R. Evans, *Cell* **57**, 1139 [1989]; M. Danielsen, L. Hinck, G. Ringold, *Cell* **57**, 1131 [1989]). "X" (solid box) indicates the DNA-binding domain (amino acids 1-87) of the *Escherichia coli* LexA protein. The "V" (hatched box) denotes a derivative of the GR N-terminal domain in which amino acids 153-406 are replaced by the transcriptional activation domain of the HSV VP16 protein (amino acids 411-490) The construct GGEc was constructed by replacing the ligand-binding domain of GGG (amino acids 528-795) with the ligand-binding domain of DHR23α (amino acids 329-878). Similarly, to construct GXEc, the ligand-binding domain of GXG (referred to as NLxC in P. J. Godowski, D. Picard and K. R. Yamamoto, *Science* **241**, 812 [1988]) was replaced with the DHR23α ligand-binding domain.

In figure 4B, the accumulation of receptor proteins in transfected cells are shown in a Westem blot detected with enzyme-linked antibodies. Whole cell extracts were prepared **48** hrs after transfection with receptor expression plasmids encoding the following fusion proteins. Lane 1, EcEcEc; lane 2, GGG; lane 3, GGEc; lane 4, VGEc; lane 5, G"E"G; lane 6, G"E"Ec; lane 7, GXG; lane 8, GXEc; lane 9, VXEc. The blots were reacted with monoclonal antibody BuGR2, that recognizes an epitope in the N-terminal domain of the rat glucocorticoid receptor (30) and then with a sheep antiserum to mouse antibody coupled to horseradish peroxidase. Positions of the molecular markers are indicated.

RNase protection analysis of transcripts induced by receptor proteins is shown in Figure 5. Total RNA was prepared from cells 48 hrs after transfection with expression plasmids encoding either DHR23α (EcEcEc), GGG, or GGEc and the reporter gene G₄M₋₇₇GO. This reporter gene contains 4 GREs fused at position -77 of an MTV promoter human growth hormone gene construct. Assays used 50 µgs of total RNA. The position of 377 base protected band for G₄M₋₇₇CO and the 294 base protected band from the intemal control gene (expressed from a CMV enhancer/promoter construct) are indicated by the closed and opened arrows, respectively.

The induction of EREs by chimeric receptors is shown in Figure 6. Cells were transfected with expression plasmids encoding either DHR23α (EcEcEc), G"E"G or G"E"Ec and E₄M₋₇₇CO, that contains 4 EREs fused to the MTV promoter. Cells were incubated for 48 hrs with or without (-) or with muristerone A (M) or dexamethasone (D). Reporter gene activity was determined as described above for Figure 2.

Illustrated in Table 1 below is the induction of a glucocorticoid receptor (GRE) responsive gene by chimeric receptors. 293 cells were transfected with effector plasmids encoding the indicated receptor proteins and the reporter gene G₄M₋₇₇CO. These transfected cells were incubated either without added hormone (-), with muristerone A (M) or with dex (D). The reporter gene activity was determined as described above for Fig. 2. The "fold induction" represents the level of expression of the reporter gene in cells incubated with hormone divided by the expression of that reporter gene in extracts from cells incubated in the absence of added ligand.

**Table 1**

| **Induction of Glucocorticoid Receptor By Chimeric receptors** | | | |
|---|---|---|---|
| Receptor | - | M | D |
| EcEcEc | 1.0 (0.2) | 0.9 (0.2) | N T |
| GGG | 1.0 (0.3) | 1.0 (0.3) | 347 |
| GGEc | 1.7 (0.3) | 798 (57) | N T |
| VGEc | 4.7 (0.8) | 3117 (240) | NT |

Illustrated in Table 2 is the transcriptional activation by receptor-LexA fusion proteins. Effector plasmids encoding the indicated receptor proteins were transfected with a reporter gene either containing (X₄C₋₃₃CO) or lacking (OC₋₃₃CO) lex-operators. "Control" indicates the cells were transfected with an "effector" plasmid which does not contain a receptor cDNA. The cells were incubated either with (+) or without (-) muristerone A. The fold induction was determined as described previously; the standard deviations are shown in parenthesis.

**Table 2**

| **Transcriptional Activation By Chimeric Receptors** | | | | |
|---|---|---|---|---|
| Receptor | X₄C₋₃₃CO | | OC₋₃₃CO | |
| | - | + | - | + |
| Control | 1.0 (0.3) | 1.1 (0.3) | 1.0 (0.2) | 1.1 (0.4) |
| EcEcEc | 1.2 (0.2) | 1.4 (0.1) | NT | NT |
| GXG | 1.0 (0.1) | 1.1 (0.2) | NT | NT |
| GXEc | 1.0 (0.2) | 44.3 (8.3) | 0.7 (0.2) | 0.9 (0.3) |
| VXEc | 4.7 (1.3) | 563 (61) | 0.8 (0.4) | 1.0 (0.2) |

While the invention has necessarily been described in conjunction with preferred embodiments, one of ordinary skill, after reading the foregoing specification, will be able to effect various changes, substitutions of equivalents, and alterations to the subject matter set forth herein, without departing from the spirit and scope thereof. Hence, the invention can be practiced in ways other than those specifically described herein. It is therefore intended that the protection granted by Letters Patent hereon be limited only by the appended claims and equivalents thereof.

## Claims

1. A method of inducing gene expression in a mammalian cell which comprises contacting an ecdysteroid with an ecdysteroid receptor polypeptide comprising a DHR23α ecdysteroid-binding domain within a mammalian cell, wherein said mammalian cell further contains a DNA binding sequence for said DHR23α ecdysteroid receptor when in combination with its ligand ecdysteroid, wherein formation of a receptor-ligand-DNA binding sequence complex induces gene expression.

2. The method of claim 1 wherein said ecdysteroid does not contain a hydroxyl group at position 25.

3. The method of claim 2 wherein said ecdysteroid contains an electron-withdrawing group at positions 5 and/or 11.

4. The method of claim 3 wherein said electron-withdrawing group is selected from the following: hydroxyl, ketone, sulfhydryl, nitrate, nitrite, fluorine, bromine, iodine and chlorine.

5. The method of claim 2 wherein said ecdysteroid is selected from the following: muristerone A, 5-dehydroxy muristerone A, 11-dehydroxy muristerone A and ponasterone.

6. The method of claim 1 wherein said DNA binding sequence further contains a DNA sequence encoding a eukaryotic gene.

7. The method of claim 6 wherein said heterologous eukaryotic gene encodes a polypeptide or nucleic acid selected from the following: cytokine, enzyme, structural polypeptide, DNA and RNA.

8. A method of producing a desired protein comprising:
a) contacting an ecdysteroid with an ecdysteroid receptor polypeptide comprising a DHR23α ecdysteroid-binding domain within a mammalian cell, wherein said mammalian cell further contains a chimeric DNA sequence comprising 1) a first DNA binding sequence having binding specificity for said DHR23α ecdysteroid receptor when in combination with its ligand ecdysteroid and 2) a second DNA sequence encoding a desired protein heterologous to said DNA binding sequence and under the transcriptional control of the ecdysteroid receptor-ligand complex; and
b) incubating said cell and producing said desired protein.

9. The method of claim 8 wherein said desired protein is selected from the following: cytokines, enzymes and structural polypeptides.

10. The method of claim 8 wherein said ecdysteroid is selected from the following: muristerone A, 5-dehydroxy muristerone A, 11-dehydroxy muristerone A and ponasterone.

11. A method of regulating gene expression in a mammalian cell line or cell culture, said mammalian cell containing a DNA sequence encoding a polypeptide heterologous to said mammalian cell and under the transcriptional control of a DHR23α ecdysteroid receptor comprising:
a) transforming said mammalian cell to express an ecdysteroid receptor fusion polypeptide comprising a DHR23α ecdysteroid-binding domain and a heterologous transactivation domain; and
b) contacting said transformed mammalian cell with an ecdysteroid that activates said DHR23α ecdysteroid receptor.

12. The method of claim 11 wherein said transactivation domain is selected from the following: herpes virus VP16 acidic activation domain, lexA, c-fos, GAL4 and c-myc.

13. The method of claim 11 wherein said ecdysteroid is selected from the following: muristerone A, 5-dehydroxy muristerone A, 11-dehydroxy muristerone A and ponasterone.

## Patentansprüche

1. Verfahren zur Induktion von Genexpression in einer Säugerzelle, welches umfaßt das Kontaktieren eines Ecdysteroids mit einem Ecdysteroid-Rezeptor-Polypeptid, umfassend eine DHR23α-Ecdysteroid-bindende Domäne, in einer Säugerzelle, wobei die Säugerzelle weiter enthält eine bindende DNA-Sequenz für den DHR23α-Ecdysteroid-Rezeptor, wenn dieser in Kombination mit seinem Liganden-Ecdysteroid vorliegt, worin die Bildung eines Rezeptor-Ligand-bindende-DNA-Sequenz-Komplexes die Genexpression induziert.

2. Verfahren nach Anspruch 1, worin das Ecdysteroid keine Hydroxylgruppe an Position 25 enthält.

3. Verfahren nach Anspruch 2, worin das Ecdysteroid eine elektronenziehende Gruppe an den Positionen 5 und/oder 11 enthält.

4. Verfahren nach Anspruch 3, worin die elektronenziehende Gruppe aus den folgenden ausgewählt ist: Hydroxyl, Keton, Sulfhydryl, Nitrat, Nitrit, Fluor, Brom, lod und Chlor.

5. Verfahren nach Anspruch 2, worin das Ecdysteroid ausgewählt ist aus den folgenden: Muristeron A, 5-Dehydroxymuristeron A, 11-Dehydroxymuristeron A und Ponasteron.

6. Verfahren nach Anspruch 1, worin die bindende DNA-Sequenz weiter eine DNA-Sequenz enthält, die für ein eukaryotisches Gen kodiert.

7. Verfahren nach Anspruch 6, worin das heterologe eukaryotische Gen kodiert für ein Polypeptid oder eine Nukleinsäure, ausgewählt aus den folgenden: Cytokin, Enzym, Struktur-Polypeptid, DNA und RNA.

8. Verfahren zur Produktion eines gewünschten Proteins, umfassend:
a) Kontaktieren eines Ecdysteroids mit einem Ecdysteroid-Rezeptor-Polypeptid, umfassend eine DHR23α-Ecdysteroid-bindende Domäne, in einer Säugerzelle, wobei die Säugerzelle weiter enthält eine chimäre DNA-Sequenz, umfassend 1) eine erste bindende DNA-Sequenz mit Bindungsspezifität für den DHR23α-Ecdysteroid-Rezeptor, wenn dieser in Kombination mit seinem Liganden-Ecdysteroid vorliegt, und 2) eine zweite DNA-Sequenz, kodierend für ein gewünschtes Protein, das heterolog zu der bindenden DNA-Sequenz ist und sich unter der transkriptionalen Kontrolle des Ecdysteroid-Rezeptor-Ligand-Komplexes befindet; und
b) Inkubation der Zelle und Produktion des gewünschten Proteins.

9. Verfahren nach Anspruch 8, worin das gewünschte Protein ausgewählt ist aus den folgenden: Cytokinen, Enzymen und Struktur-Polypeptiden.

10. Verfahren nach Anspruch 8, worin das Ecdysteroid ausgewählt ist aus den folgenden: Muristeron A, 5-Dehydroxymuristeron A, 11-Dehydroxymuristeron A und Ponasteron.

11. Verfahren zur Regulation der Genexpression in einer Säuger-Zellinie oder -Zellkultur, wobei die Säugerzelle eine DNA-Sequenz enthält, die für ein Polypeptid kodiert, das heterolog zu der Säugerzelle ist und sich unter der transkriptionalen Kontrolle eines DHR23α-Ecdysteroid-Rezeptors befindet, umfassend:
a) Transformation der Säugerzelle, um ein Ecdysteroid-Rezeptor-Fusionspolypeptid, welches eine DHR23α-Ecdysteroid-bindende Domäne und eine heterologe Transaktivierungsdomäne umfaßt, zu exprimieren; und
b) Kontaktieren der transformierten Säugerzelle mit einem Ecdysteroid, welches den DHR23α-Ecdysteroid-Rezeptor aktiviert.

12. Verfahren nach Anspruch 11, wobei die Transaktivierungsdomäne ausgewählt ist aus den folgenden: saure Aktivierungsdomäne des Herpesvirus VP16, lexA, c-fos, GAL4 und c-myc.

13. Verfahren nach Anspruch 11, worin das Ecdysteroid ausgewählt ist aus den folgenden: Muristeron A, 5-Dehydroxymuristeron A, 11-Dehydroxymuristeron A und Ponasteron.

## Revendications

1. Procédé d'induction de l'expression de gènes dans des cellules de mammifère qui comprend la mise en contact d'un ecdystéroïde avec un polypeptide récepteur d'ecdystéroïde comprenant un domaine de liaison d'ecdystéroïde DHR23α au sein d'une cellule de mammifère, dans lequel ladite cellule de mammifère contient aussi une séquence de liaison ADN pour ledit récepteur d'ecdystéroïde DHR23α lorsqu'elle est en combinaison avec son ligand ecdystéroïde, dans lequel la formation d'un complexe de séquence de liaison récepteur-ligand-ADN induit l'expression des gênes.

2. Procédé selon la revendication 1, dans lequel ledit ecdystéroïde ne contient pas de groupe hydroxyle en position 25.

3. Procédé selon la revendication 2, dans lequel ledit ecdystéroïde contient un groupe capteur d'électrons en position 5 et/ou en position 11.

4. Procédé selon la revendication 3, dans lequel ledit groupe capteur d'électrons est choisi au sein de l'ensemble suivant: hydroxyle, cétone, sulfhydryl, nitrate, nitrite, fluor, brome, iode et chlore

5. Procédé selon la revendication 2, dans lequel ledit ecdystéroïde est choisi parmi les suivants : muristérone A, 5-déshydroxymuristérone A, 11-déshydroxymuristérone A et ponastérone.

6. Procédé selon la revendication 1, dans lequel ladite séquence de liaison ADN contient aussi une séquence d'ADN encodant un gêne eucaryote.

7. Procédé selon la revendication 6, dans lequel le gène eucaryote hétérologue encode un polypeptide ou un acide nucléique choisi parmi les suivants : cytokine, enzyme, polypeptide structural, ADN et ARN.

8. Procédé de production d'une protéine désirée comprenant :
(a) la mise en contact d'un polypeptide récepteur d'ecdystéroïde comprenant un domaine de liaison d'ecdystéroïde DHR23α au sein d'une cellule de mammifère, dans lequel ladite cellule de mammifère contient aussi une séquence d'ADN chimérique comprenant (1) une première séquence de liaison d'ADN ayant une spécificité de liaison pour ledit récepteur d'ecdystéroïde DHR23α lorsqu'elle est en en combinaison avec son ligand ecdystéroïde et (2) une seconde séquence d'ADN encodant une protéine désirée hétérologue vis-à-vis de la séquence d'ADN de liaison et sous le contrôle transcriptionnel du complexe récepteur d'ecdystéroïde-ligand ; et
(b) l'incubation de ladite cellule et la production de ladite protéine.

9. Procédé selon la revendication 8, dans lequel ladite protéine est choisie parmi les suivantes: cytokines, enzymes et polypeptides structuraux.

10. Procédé selon la revendication 6, dans lequel ledit ecdystéroïde est choisi parmi les suivants : muristérone A, 5-déshydroxymuristérone A, 11-déshydroxymuristérone A et ponastérone.

11. Procédé de régulation de l'expression de gènes dans une lignée cellulaire ou une culture de cellule de mammifère, ladite cellule de mammifère contenant une séquence d'ADN encodant un polypeptide hétérologue vis-à-vis de ladite cellule de mammifère et sous le contrôle transcriptionnel d'un récepteur d'ecdystéroïde DHR23α comprenant:
(a) la transformation de ladite cellule de mammifère pour exprimer un polypeptide de fusion récepteur d'ecdystéroïde DHR23α comprenant un domaine de liaison d'ecdystéroïde DHR23α et un domaine de transcription hétérologue ; et
(b) la mise en contact de ladite cellule de mammifère transformée avec un ecdystéroïde qui active ledit récepteur d'ecdystéroïde DHR23α.

12. Procédé selon la revendication 11, dans lequel ledit domaine de transactivation est choisi parmi les suivants: domaine d'activation acide du virus de l'herpès VP16, lexA, c-fos, GAL4 et c-myc.

13. Procédé selon la revendication 11, dans lequel ledit ecdystéroïde est choisi parmi les suivants : muristérone A, 5-déshydroxymuristérone A, 11-déshydroxymuristérone A et ponastérone.


### SEQUENCE LISTING

(1) GENERAL INFORMATION:
   (i) APPLICANT: Genentech, Inc.
   (ii) TITLE OF INVENTION: Ecdysteroid Dependent Regulation of Genes In Mammalian Cells
   (iii) NUMBER OF SEQUENCES: 1
   (iv) CORRESPONDENCE ADDRESS:
      (A) ADDRESSEE: Genentech, Inc.
      (B) STREET: 460 Point San Bruno Blvd
      (C) CITY: South San Francisco
      (D) STATE: California
      (E) COUNTRY: USA
      (F) ZIP: 94080
   (v) COMPUTER READABLE FORM:
      (A) MEDIUM TYPE: 5.25 inch, 360 Kb floppy disk
      (B) COMPUTER: IBM PC compatible
      (C) OPERATING SYSTEM: PC-DOS/MS-DOS
      (D) SOFTWARE: patin (Genentech)
   (vii) CURRENT APPLICATION DATA:
      (A) APPLICATION NUMBER:
      (B) FILING DATE: 03-AUG-1992
      (C) CLASSIFICATION:
   (viii) ATTORNEY/AGENT INFORMATION:
      (A) NAME: Fitts, Renee A.
      (B) REGISTRATION NUMBER: 35,136
      (C) REFERENCE/DOCKET NUMBER: 607
   (ix) TELECOMMUNICATION INFORMATION:
      (A) TELEPHONE: 415/225-1489
      (B) TELEFAX: 415/952-9881
      (C) TELEX: 910/371-7168
(2) INFORMATION FOR SEQ ID NO:1:
   (i) SEQUENCE CHARACTERISTICS:
      (A) LENGTH: 2970 bases
      (B) TYPE: nucleic acid
      (C) STRANDEDNESS: single
      (D) TOPOLOGY: linear
   (xi) SEQUENCE DESCRIPTION: SEQ ID NO:1:

AAGCTTCTTG TCCCCAGCCG ACGCTAAGTG AACGGAAAAC GGCCACAAAA 50

CGGCGACTAT CGGCTGCCAG AGG ATG AAG CGG CGC TGG TCG 91
Met Lys Arg Arg Trp Ser
1               5

AAC AAC GGC GGC TTC ATG CGC CTA CCG GAG GAG TCG TCC 130
Asn Asn Gly Gly Phe Met Arg Leu Pro Glu Glu Ser Ser
            10                  15

TCG GAG GTC ACG TCC TCC TCG AAC GGG CTC GTC CTG CCC 169
Ser Glu Val Thr Ser Ser Ser Asn Gly Leu Val Leu Pro
20                  25                  30

TCG GGG GTG AAC ATG TCG CCC TCG TCG CTG GAC TCG CAC 208
Ser Gly Val Asn Met Ser Pro Ser Ser Leu Asp Ser His
        35                  40                  45

GAC TAT TGC GAT CAG GAC CTT TGG CTC TGC GGC AAC GAG 247
Asp Tyr Cys Asp Gln Asp Leu Trp Leu Cys Gly Asn Glu
                50                  55

TCC GGT TCG TTT GGC GGC TCC AAC GGC CAT GGC CTA AAT 286
Ser Gly Ser Phe Gly Gly Ser Asn Gly His Gly Leu Asn
    60                  65                  70

CAG CAG CAG CAG AGC GTC ATC ACG CTG GCC ATG CAC GGG 325
Gln Gln Gln Gln Ser Val Ile Thr Leu Ala Met His Gly
            75                  80

TGC TCC AGC ACT CTG CCC GCG CAG ACA ACC ATC ATT CCG 364
Cys Ser Ser Thr Leu Pro Ala Gln Thr Thr Ile Ile Pro
85                  90                  95

ATC AAC GGC AAC GCG AAT GGG AAT GGA GGC TCC ACC AAT 403
Ile Asn Gly Asn Ala Asn Gly Asn Gly Gly Ser Thr Asn
        100                 105                 110

GGC CAA TAT GTG CCG GGT GCC ACC AAT CTG GGA GCG TTG 442
Gly Gln Tyr Val Pro Gly Ala Thr Asn Leu Gly Ala Leu
                115                 120

GCC AAC GGG ATG CTC AAT GGG GGC CTC AAT GGA ATG CAG 481
Ala Asn Gly Met Leu Asn Gly Gly Leu Asn Gly Met Gln
    125                 130                 135

CAA CAG ATT CAG AAT GGC CAC GGC CTC ATC AAC TCC ACA 520
Gln Gln Ile Gln Asn Gly His Gly Leu Ile Asn Ser Thr
            140                 145

ACG CCC TCA ACG CCG ACC ACC CCG CTC CAC CTT CAG CAG 559
Thr Pro Ser Thr Pro Thr Thr Pro Leu His Leu Gln Gln
150                 155                 160

AAC CTG GGG GGC GCG GGC GGC GGC GGT ATC GGG GGA ATG 598
Asn Leu Gly Gly Ala Gly Gly Gly Gly Ile Gly Gly Met
        165                 170                 175

GGT ATT CTT CAC CAC GCG AAT GGC ACC CCA AAT GGC CTT 637
Gly Ile Leu His His Ala Asn Gly Thr Pro Asn Gly Leu
                180                 185

ATC GGA GTT GTG GGA GGC GGC GGC GGA GTA GGT CTT GGA 676
Ile Gly Val Val Gly Gly Gly Gly Gly Val Gly Leu Gly
    190                 195                 200

GTA GGC GGA GGC GGA GTG GGA GGC CTG GGA ATG CAG CAC 715
Val Gly Gly Gly Gly Val Gly Gly Leu Gly Met Gln His
            205                 210

ACA CCC CGA AGC GAT TCG GTG AAT TCT ATA TCT TCA GGT 754
Thr Pro Arg Ser Asp Ser Val Asn Ser Ile Ser Ser Gly
215                 220                 225

CGC GAT GAT CTC TCG CCT TCG AGC AGC TTG AAC GGA TAC 793
Arg Asp Asp Leu Ser Pro Ser Ser Ser Leu Asn Gly Tyr
        230                 235                 240

TCG GCG AAC GAA AGC TGC GAT GCG AAG AAG AGC AAG AAG 832
Ser Ala Asn Glu Ser Cys Asp Ala Lys Lys Ser Lys Lys
                245                 250

GGA CCT GCG CCA CGG GTG CAA GAG GAG CTG TGC CTG GTT 871
Gly Pro Ala Pro Arg Val Gln Glu Glu Leu Cys Leu Val
    255                 260                 265

TGC GGC GAC AGG GCC TCC GGC TAC CAC TAC AAC GCC CTC 910
Cys Gly Asp Arg Ala Ser Gly Tyr His Tyr Asn Ala Leu
            270                 275

ACC TGT GAG GGC TGC AAG GGG TTC TTT CGA CGC AGC GTT 949
Thr Cys Glu Gly Cys Lys Gly Phe Phe Arg Arg Ser Val
280                 285                 290

ACG AAG AGC GCC GTC TAC TGC TGC AAG TTC GGG CGC GCC 988
Thr Lys Ser Ala Val Tyr Cys Cys Lys Phe Gly Arg Ala
        295                 300                 305

TGC GAA ATG GAC ATG TAC ATG AGG CGA AAG TGT CAG GAG 1027
Cys Glu Met Asp Met Tyr Met Arg Arg Lys Cys Gln Glu
                310                 315

TGC CGC CTG AAA AAG TGC CTG GCC GTG GGT ATG CGG CCG 1066
Cys Arg Leu Lys Lys Cys Leu Ala Val Gly Met Arg Pro
    320                 325                 330

GAA TGC GTC GTC CCG GAG AAC CAA TGT GCG ATG AAG CGG 1105
Glu Cys Val Val Pro Glu Asn Gln Cys Ala Met Lys Arg
            335                 340

CGC GAA AAG AAG GCC CAG AAG GAG AAG GAC AAA ATG ACC 1144
Arg Glu Lys Lys Ala Gln Lys Glu Lys Asp Lys Met Thr
345                 350                 355

ACT TCG CCG AGC TCT CAG CAT GGC GGC AAT GGC AGC TTG 1183
Thr Ser Pro Ser Ser Gln His Gly Gly Asn Gly Ser Leu
        360                 365                 370

GCC TCT GGT GGC GGC CAA GAC TTT GTT AAG AAG GAG ATT 1222
Ala Ser Gly Gly Gly Gln Asp Phe Val Lys Lys Glu Ile
                375                 380

CTT GAC CTT ATG ACA TGC GAG CCG CCC CAG CAT GCC ACT 1261
Leu Asp Leu Met Thr Cys Glu Pro Pro Gln His Ala Thr
    385                 390                 395

ATT CCG CTA CTA CCT GAT GAA ATA TTG GCC AAG TGT CAA 1300
Ile Pro Leu Leu Pro Asp Glu Ile Leu Ala Lys Cys Gln
            400                 405

GCG CGC AAT ATA CCT TCC TTA ACG TAC AAT CAG TTG GCC 1339
Ala Arg Asn Ile Pro Ser Leu Thr Tyr Asn Gln Leu Ala
410                 415                 420

GTT ATA TAC AAG TTA ATT TGG TAC CAG GAT GGC TAT GAG 1378
Val Ile Tyr Lys Leu Ile Trp Tyr Gln Asp Gly Tyr Glu
        425                 430                 435

CAG CCA TCT GAA GAG GAT CTC AGG CGT ATA ATG AGT CAA 1417
Gln Pro Ser Glu Glu Asp Leu Arg Arg Ile Met Ser Gln
                440                 445

CCC GAT GAG AAC GAG AGC CAA ACG GAC GTC AGC TTT CGG 1456
Pro Asp Glu Asn Glu Ser Gln Thr Asp Val Ser Phe Arg
    450                 455                 460

CAT ATA ACC GAG ATA ACC ATA CTC ACG GTC CAG TTG ATT 1495
His Ile Thr Glu Ile Thr Ile Leu Thr Val Gln Leu Ile
            465                 470

GTT GAG TTT GCT AAA GGT CTA CCA GCG TTT ACA AAG ATA 1534
Val Glu Phe Ala Lys Gly Leu Pro Ala Phe Thr Lys Ile
475                 480                 485

CCC CAG GAG GAC CAG ATC ACG TTA CTA AAG GCC TGC TCG 1573
Pro Gln Glu Asp Gln Ile Thr Leu Leu Lys Ala Cys Ser
        490                 495                 500

TCG GAG GTG ATG ATG CTG CGT ATG GCA CGA CGC TAT GAC 1612
Ser Glu Val Met Met Leu Arg Met Ala Arg Arg Tyr Asp
                505                 510

CAC AGC TCG GAC TCA ATA TTC TTC GCG AAT AAT AGA TCA 1651
His Ser Ser Asp Ser Ile Phe Phe Ala Asn Asn Arg Ser
    515                 520                 525

TAT ACG CGG GAT TCT TAC AAA ATG GCC GGA ATG GCT GAT 1690
Tyr Thr Arg Asp Ser Tyr Lys Met Ala Gly Met Ala Asp
            530                 535

AAC ATT GAA GAC CTG CTG CAT TTC TGC CGC CAA ATG TTC 1729
Asn Ile Glu Asp Leu Leu His Phe Cys Arg Gln Met Phe
540                 545                 550

TCG ATG AAG GTG GAC AAC GTC GAA TAC GCG CTT CTC ACT 1768
Ser Met Lys Val Asp Asn Val Glu Tyr Ala Leu Leu Thr
        555                 560                 565

GCC ATT GTG ATC TTC TCG GAC CGG CCG GGC CTG GAG AAG 1807
Ala Ile Val Ile Phe Ser Asp Arg Pro Gly Leu Glu Lys
                570                 575

GCC CAA CTA GTC GAA GCG ATC CAG AGC TAC TAC ATC GAC 1846
Ala Gln Leu Val Glu Ala Ile Gln Ser Tyr Tyr Ile Asp
    580                 585                 590

ACG CTA CGC ATT TAT ATA CTC AAC CGC CAC TGC GGC GAC 1885
Thr Leu Arg Ile Tyr Ile Leu Asn Arg His Cys Gly Asp
            595                 600

TCA ATG AGC CTC GTC TTC TAC GCA AAG CTG CTC TCG ATC 1924
Ser Met Ser Leu Val Phe Tyr Ala Lys Leu Leu Ser Ile
605                 610                 615

CTC ACC GAG CTG CGT ACG CTG GGC AAC CAG AAC GCC GAG 1963
Leu Thr Glu Leu Arg Thr Leu Gly Asn Gln Asn Ala Glu
        620                 625                 630

ATG TGT TTC TCA CTA AAG CTC AAA AAC CGC AAA CTG CCC 2002
Met Cys Phe Ser Leu Lys Leu Lys Asn Arg Lys Leu Pro
                635                 640

AAG TTC CTC GAG GAG ATC TGG GAC GTT CAT GCC ATC CCG 2041
Lys Phe Leu Glu Glu Ile Trp Asp Val His Ala Ile Pro
    645                 650                 655

CCA TCG GTC CAG TCG CAC CTT CAG ATT ACC CAG GAG GAG 2080
Pro Ser Val Gln Ser His Leu Gln Ile Thr Gln Glu Glu
            660                 665

AAC GAG CGT CTC GAG CGG GCT GAG CGT ATG CGG GCA TCG 2119
Asn Glu Arg Leu Glu Arg Ala Glu Arg Met Arg Ala Ser
670                 675                 680

GTT GGG GGC GCC ATT ACC GCC GGC ATT GAT TGC GAC TCT 2158
Val Gly Gly Ala Ile Thr Ala Gly Ile Asp Cys Asp Ser
        685                 690                 695

GCC TCC ACT TCG GCG GCG GCA GCC GCG GCC CAG CAT CAG 2197
Ala Ser Thr Ser Ala Ala Ala Ala Ala Ala Gln His Gln
                700                 705

CCT CAG CCT CAG CCC CAG CCC CAA CCC TCC TCC CTG ACC 2236
Pro Gln Pro Gln Pro Gln Pro Gln Pro Ser Ser Leu Thr
    710                 715                 720

CAG AAC GAT TCC CAG CAC CAG ACA CAG CCG CAG CTA CAA 2275
Gln Asn Asp Ser Gln His Gln Thr Gln Pro Gln Leu Gln
            725                 730

CCT CAG CTA CCA CCT CAA CTG CAA GGT CAA CTG CAA CCC 2314
Pro Gln Leu Pro Pro Gln Leu Gln Gly Gln Leu Gln Pro
735                 740                 745

CAG CTC CAA CCA CAG CTT CAG ACG CAA CTC CAG CCA CAG 2353
Gln Leu Gln Pro Gln Leu Gln Thr Gln Leu Gln Pro Gln
        750                 755                 760

ATT CAA CCA CAG CCA CAG CTC CTT CCC GTC TCC GCT CCC 2392
Ile Gln Pro Gln Pro Gln Leu Leu Pro Val Ser Ala Pro
                765                 770

GTG CCC GCC TCC GTA ACC GCA CCT GGT TCC TTG TCC GCG 2431
Val Pro Ala Ser Val Thr Ala Pro Gly Ser Leu Ser Ala
    775                 780                 785

GTC AGT ACG AGC AGC GAA TAC ATG GGC GGA AGT GCG GCC 2470
Val Ser Thr Ser Ser Glu Tyr Met Gly Gly Ser Ala Ala
            790                 795

ATA GGA CCC ATC ACG CCG GCA ACC ACC AGC AGT ATC ACG 2509
Ile Gly Pro Ile Thr Pro Ala Thr Thr Ser Ser Ile Thr
800                 805                 810

GCT [?][?]C GTT ACC GCT AGC TCC ACC ACA TCA GCG GTA CCG 2548
Ala Ala Val Thr Ala Ser Ser Thr Thr Ser Ala Val Pro
        815                 820                 825

ATG GGC AAC GGA GTT GGA GTC GGT GTT GGG GTG GGC GGC 2587
Met Gly Asn Gly Val Gly Val Gly Val Gly Val Gly Gly
                830                 835

AAC GTC AGC ATG TAT GCG AAG CCC CAG ACG GCG ATG GCC 2626
Asn Val Ser Met Tyr Ala Lys Pro Gln Thr Ala Met Ala
    840                 845                 850

TTG ATG GGT GTA GCC CTG CAT TCG CAC CAA GAG CAG CTT 2665
Leu Met Gly Val Ala Leu His Ser His Gln Glu Gln Leu
            855                 860

ATC GGG GGA GTG GCG GTT AAG TCG GAG CAC TCG ACG ACT 2704
Ile Gly Gly Val Ala Val Lys Ser Glu His Ser Thr Thr
865                 870                 875

GCA TAG CAGGCGCAGA GTCAGCTCCA CCAACATCAC CACCACAACA 2750
Ala
878

TCGACGTCCT GCTGGAGTAG AAAGCGCAGC TGAACCCACA CAGACATAGG 2800

GGAAATGGGG AAGTTCTCTC CAGAGAGTTC GAGCCGAACT AAATAGTAAA 2850

AAGTGAATAA TTAATGGACA AGCGTAAAAT GCAGTTATTT AGTCTTAAGC 2900

CTGCAAATAT TACCTATTAT TCATACAAAT TAACATATAA TACAGCCTAT 2950

TAACAATTAC GCTAAAGCTT 2970